# FASCICULE DE BREVET EUROPEEN

(11) **EP 1 233 786 B1**
(45) Date de publication et mention de la délivrance du brevet: **18.02.2004**
(21) Numéro de dépôt: 00974645.4
(22) Date de dépôt: 03.11.2000
(51) Int. Cl.: A61K 45/06, A61P 3/00, A61P 9/00, A61P 11/00, A61P 17/00, A61P 19/00, A61P 25/00, A61P 27/00, A61P 29/00, A61P 31/00, A61P 33/00, A61P 35/00, A61P 37/00

(54) **PRODUIT COMPRENANT AU MOINS UNE SUBSTANCE INHIBITRICE DES NO SYNTHASES EN ASSOCIATION AVEC AU MOINS UNE SUBSTANCE INHIBITRICE DES PHOSPHOLIPASES A2 POUR LA PREPARATION D'UN MEDICAMENT**
ZUSAMMENSETZUNG DIE MINDESTENS EIN STICKSTOFFMONOXID-SYNTHASE INHIBITOR UND MINDESTENS EIN PHOSPHOLIPASE A2 INHIBITOR ENTHÄLT ZUR HERSTELLUNG EINES MEDIKAMENTES
PRODUCT COMPRISING AT LEAST A NO SYNTHASE INHIBITING SUBSTANCE ASSOCIATED WITH AT LEAST A PHOSPHOLIPASE A2 INHIBITING SUBSTANCE FOR THE PREPARATION OF A MEDICAMENT

(30) Priorité: 05.11.1999 FR 9913859
(43) Date de publication de la demande: 28.08.2002
(73) Titulaire: SOCIETE DE CONSEILS DE RECHERCHES ET D'APPLICATIONS SCIENTIFIQUES (S.C.R.A.S.), 75016 Paris (FR)
(72) Inventeur: AUGUET, Michel, F-91120 Palaiseau (FR); CHABRIER DE LASSAUNIERE, Pierre-Etienne, F-75016 Paris (FR)
(74) Mandataire: Bourgouin, André
(86) Numéro de dépôt international: PCT/FR2000/003066
(87) Numéro de publication internationale: WO 2001/032216

(56) Documents cités:
- HOLSCHER, CHRISTIAN ET AL: "Inhibitors of PLA2 [phospholipase A2] and NO synthase cooperate in producing amnesia of a spatial task" NEUROREPORT (1995), 6(5), 730-2, 1995, XP000923049
- NISHIBORI, M. ET AL: "Effect of histamine and its analogs on cyclic AMP level in canine parotid gland" ADV. BIOSCI. (1982), 33(ADV. HISTAMINE RES.), 203-9, XP000923046

## Description

L'invention concerne la préparation d'un médicament comprenant au moins une substance inhibitrice de NO synthases en association avec au moins une substance inhibitrice de phospholipases A2, sous forme séparée ou en combinaison, pour une utilisation thérapeutique simultanée, séparée ou étalée dans le temps dans le traitement de pathologies dans lesquelles le monoxyde d'azote et/ou les phospholipases A2 sont impliquées. L'invention concerne également une composition pharmaceutique comprenant, à titre de principe actif, au moins une substance inhibitrice de NO synthases et au moins une substance inhibitrice de phospholipases A2, et éventuellement un support pharmaceutiquement acceptable.

Une composition pharmaceutique préparée selon l'invention est intéressante dans le traitement de pathologies où le monoxyde d'azote et les phospholipases A2 sont impliquées, sélectionnées parmi :
- les maladies prolifératives, inflammatoires et oedémateuses comme, par exemple, l'athérosclérose, la resténose, l'hypertension pulmonaire, la glomérulonéphrite, l'hypertension portale, le psoriasis, l'arthrose et l'arthrite rhumatoïde, les fibroses, les amyloïdoses, les inflammations du système gastrointestinal (colite, maladie de Crohn, la pancréatite) ou du système pulmonaire et des voies aériennes (asthme, sinusites, syndrome de détresse respiratoire), la rhinite allergique ;
- les troubles cardiovasculaires et cérébrovasculaires comprenant, par exemple, la migraine, l'hypertension artérielle, le choc septique, les infarctus cardiaques ou cérébraux d'origine ischémique ou hémorragique, les ischémies et les thromboses ;
- les troubles du système nerveux central ou périphérique comme, par exemple, les maladies neurodégénératives où l'on peut notamment citer les infarctus cérébraux, les démences séniles, y compris la maladie d'Alzheimer, la chorée de Huntington, la maladie de Parkinson, la maladie de Creutzfeld Jacob, les maladies dues aux prions, la sclérose latérale amyotrophique mais aussi la douleur, les traumatismes cérébraux ou de la moëlle épinière, l'addiction aux opiacés, à l'alcool et aux substances induisant une accoutumance ;
- les dermatites, radiations solaires (UVA, UVB) ;
- les transplantations d'organes ;
- les maladies autoimmunes et virales comme par exemple le lupus, le sida, les infections parasitaires et virales, le diabète, la sclérose en plaques, les myopathies ;
- le cancer et les maladies ophtalmiques ;
- et plus généralement toutes les pathologies caractérisées par une production ou une hyperactivation de monoxyde d'azote et/ou de phospholipase A2.

Dans l'ensemble de ces pathologies, il existe des évidences expérimentales démontrant l'implication du manoxyde d'azote (*J. Med. Chem*. (1995) **38,** 4343-4362; *Clin. Exp. Immunol*. (1998) **113,** 147-156) et des phospholipases A2 *(Trends Pharmac. Sci*. (1999), **20,** 162-16170; *Brain Res. Bull.* (1999), **49,** 139-153). C'est le cas notamment de l'inflammation expérimentale de la patte de rat qui illustre l'invention *(Agents Actions* (1989) **26,** 292-300 ; *Immunopharmacology* (1993), **26,** 1-9 ; *Br. J. Pharmacol*. (1998), **123,** 1119-1126).

Dans ce contexte, les médicaments pouvant inhiber la formation du monoxyde d'azote ou inhiber les phospholipases A2 peuvent apporter des effets bénéfiques. Aucune association de ces deux principes actifs, à savoir un inhibiteur de NO synthase et un inhibiteur des phospholipases A2, n'a été réalisée. Comme cela est exposé dans la partie expérimentale, l'association d'un inhibiteur de NO synthases et d'un inhibiteur des phospholipases A2, principes actifs agissant par des mécanismes différents, provoque un effet thérapeutique inattendu de ces principes actifs, à savoir qu'ils agissent de manière synergique. En effet, ceux-ci, administrés à des doses subactives (c'est-à-dire à des doses qui ne produisent pas par elles-mêmes d'effet thérapeutique), produisent, lorsqu'ils sont associés, un effet thérapeutique hautement significatif.

L'avantage de cette association est de diminuer de façon importante les doses de chacun des principes actifs et ainsi de diminuer considérablement leurs effets indésirables tout en gagnant de l'efficacité thérapeutique. Cette invention est particulièrement bien illustrée dans un modèle pathologique expérimental d'inflammation de la patte de rat par la carragénine.

L'invention a tout d'abord pour objet la préparation d'un médicament comprenant au moins une substance inhibitrice de NO synthases en association au moins une substance inhibitrice de phospholipases A2, sous forme séparée ou en combinaison, pour une utilisation thérapeutique simultanée, séparée ou étalée dans le temps dans le traitement de pathologies dans lesquelles le monoxyde d'azote et/ou les phospholipases A2 sont impliquées. De préférence, ledit produit comprendra une substance inhibitrice de NO synthèse en association avec une substance inhibitrice des phospholipases A2.

Par inhibiteur de NO synthases, il faut entendre tout inhibiteur spécifique ou non spécifique de l'une de ses isoformes qu'elle soit constitutive (neuronale ou endothéliale) ou inductible (*J. Med. Chem.* (1995) **38,** 4343-4362). De préférence, les inhibiteurs de NO synthases utilisés pour la présente invention seront des inhibiteurs des formes neuronale ou inductible des NO synthases.

Par inhibiteur des phospholipases A2, il faut entendre tout inhibiteur spécifique ou non spécifique de l'une des formes de phospholipases A2 sécrétée ou cellulaire (*Médecine*/*Sciences* (1995) **12,** 323-332; *Trends Pharmac. Sci*. (1999) **20,** 16162-16170; *Brain Res. Bull.* (1999) **49**, 139-153).

L'inhibiteur de NO synthases et l'inhibiteur des phospholipases A2 peuvent se présenter sous forme séparée ou sous forme combinée en formant un sel. De préférence, le sel est formé à partir d'un dérivé de la substance inhibitrice de NO synthase contenant au moins un groupe basique et d'un dérivé inhibiteur des phospholipases A2 contenant au moins un groupe acide. Ainsi des sels peuvent être formés, selon les méthodes connues de l'homme du métier, à partir des inhibiteurs de NO synthase comme par exemple les amidines, les guanidines, les pyridines ou les pipéridines telles que définies ci-après, et des inhibiteurs des phospholipases A2 comme par exemple la mépacrine, l'acide aristolochique, les acides acylpyrroldicarboxyliques ou acylindoldicarboxyliques tels que définis ci-après. Ces substances peuvent être naturelles ou synthétiques.

L'invention a également pour objet la préparation d'un médicament comprenant au moins une substance inhibitrice de NO synthase en association avec au moins un dérivé inhibiteur des phospholipases A2, sous forme séparée ou en combinaison, pour une utilisation thérapeutique simultanée, séparée ou étalée dans le temps dans le traitement de pathologies dans lesquelles le monoxyde d'azote et/ou les phospholipases A2 sont impliquées, telles que notamment les troubles cardiovasculaires et cérébrovasculaires, les troubles du système nerveux central ou périphérique, les maladies prolifératives et inflammatoires, les transplantations d'organes, les maladies autoimmunes et virales, le cancer, et plus généralement toutes les pathologies caractérisées soit par une production excessive de monoxyde d'azote et/ou des phospholipases A2, soit par un dysfonctionnement lié au monoxyde d'azote et/ou aux phospholipases A2.

Parmi les inhibiteurs de NO synthases, on peut définir les composés de type amino-acide et non amino-acide. Les inhibiteurs de NO synthases de type amino-acide peuvent être des composés tels que décrits dans les demandes WO 95/00505, WO 94/12163, WO 96/06076 et le demande EP 230037, ou bien des dérivés de la L-arginine, de l'omithine ou de la lysine tels que décrits dans les demandes PCT WO 93/24126, WO 95/01972, WO 95/24382, WO 95/09619 et WO 95/22968.

Les inhibiteurs de NO synthases de type non amino-acide, peuvent être des composés de la familles des guanidines, des isothiourées, des nitro- ou cyano-aryles, des amino-pyridines ou amino-pyrimidines, des amidines, des indazoles ou des imidazoles.

Les dérivés de guanidines inhibiteurs de NO synthases peuvent être les composés tels que définis dans les demandes PCT WO 95/28377, WO 91/04023, WO 94/21621, WO 96/18607 et WO 96/18608.

Les isothiourées inhibiteurs de NO synthases peuvent être les composés tels que définis dans les demandes PCT WO 95/09619, WO 96/09286, WO 94/12165, WO 96/14842, WO 96/18607, WO 96/18608, WO 96/09286 et les demandes EP 717040 et EP 718294

Les nitro- ou cyano-aryles inhibiteurs de NO synthases peuvent être les composés tels que définis dans la demande PCT WO 94/12163.

Les amino-pyridines ou amino-pyrimidines inhibiteurs de NO synthases peuvent être les composés tels que définis dans les demandes PCT WO 94/14780, WO 96/18616 et WO 96/18617.

Les amidines inhibiteurs de NO synthase peuvent être les composés tels que définis dans les demandes PCT WO 95/11014, WO 96/01817, WO 95/05363, WO 95/11231, WO 96/14844 et WO 96/19440 ou des composés tels que le N-phényl-2-thiophèneearboximidamide.

Par ailleurs, les inhibiteurs de NO synthases peuvent également être à la fois des inhibiteurs de NO synthases (NOS) et des piégeurs des formes réactives de l'oxygène (ROS pour *Reactive Oxygen Species).* La demanderesse a, la première, décrit de tels inhibiteurs dans les demandes de brevet PCT WO 98/42696, WO 98/58934, WO 00/02860, WO 00/17190 et WO 00/17191, (d'autres demandes de brevet non publiées décrivant des composés présentant de telles activités pharmacologiques ont aussi été déposées par la demanderesse et les composés qui y sont décrits peuvent aussi être utilisés selon la présente invention). Ces composés présentent également une synergie dans le modèle pathologique expérimental d'inflammation de la patte de rat par la carragénine, tout en conservant les avantages déjà exposés dans les demandes PCT WO 98/42696 et WO 98/58934, WO 00/02860, WO 00/17190 et WO 00/17191.

Les indazoles inhibiteurs de NO synthases peuvent être des composés de formule générale I_{A} 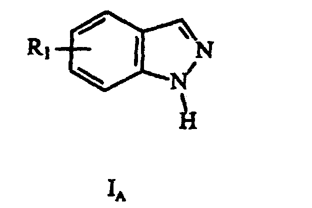 dans laquelle R₁ représente un ou plusieurs substituants choisis parmi l'atome d'hydrogène et les radicaux nitro, halo, alkyle ou alkoxy ; ou bien des sels pharmaceutiquement acceptables des composés de formule générale I_{A}.

Les imidazoles inhibiteurs de NO synthases peuvent être les composés de formule générale II_{A} 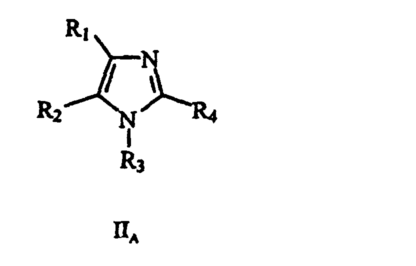 dans laquelle
R₁ et R₂ représentent, indépendemment, un atome d'hydrogène ou le radical halo, hydroxy, amino, alkyle ou alkoxy, ou R₁ et R₂ sont liés ensemble et forment un radical phényle condensé avec le cycle imidazole, ledit radical phényle étant éventuellement substitué par un ou plusieurs substituants choisis parmi les radicaux hydroxy, trifluorométhyle, halo, carboxy, alkyle, alkoxy ou alkényle ;
R₃ représente un atome d'hydrogène ou un radical alkyle, amino, alkylamino ou phényle, ledit radical phényle étant éventuellement substitué par un ou plusieurs substituants choisis parmi les radicaux hydroxy, trifluorométhyle, halo, carboxy, alkyle, alkoxy ou alkényle ;
et R₄ représente un atome d'hydrogène ou un radical alkyle, amino ou alkylamino ; ou bien des sels phannaceutiquement acceptables des composés de formule générale II_{A}.

Les composés inhibiteurs de NO synthases et de ROS peuvent notamment être les composés de formule générale III_{A} 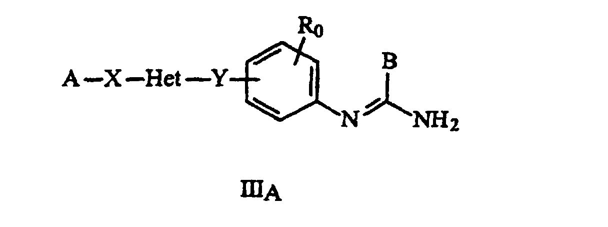 dans laquelle
R₀ représente H ou un radical alkyle;
A est un radical 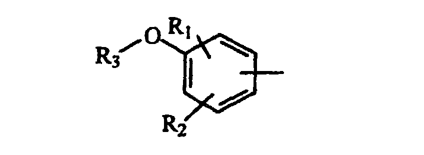 dans lequel R₁ et R₂ représentent, indépendamment, un atome d'hydrogène, un halogène, le groupe OH, un radical alkyle linéaire ou ramifié ayant de 1 à 6 atomes de carbone, un radical alkoxy linéaire ou ramifié ayant de 1 à 6 atomes de carbone,
et R₃ représente un atome d'hydrogène, un radical alkyle linéaire ou ramifié ayant de 1 à 6 atomes de carbone ou un radical -COR₄, R₄ représentant un radical alkyle ayant de 1 à 6 atomes de carbone,
ou un radical 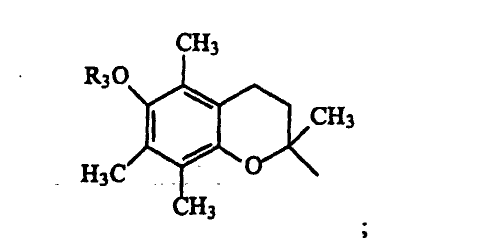 dans lequel R₃ représente un atome d'hydrogène, un radical alkyle linéaire ou ramifié ayant de 1 à 6 atomes de carbone ou un radical -COR₄, R₄ représentant un radical alkyle ayant de 1 à 6 atomes de carbone ;
B représente un radical alkyle linéaire ou ramifié ayant de 1 à 6 atomes de carbone, phényle, pyridinyle ou un hétérocycle à 5 chaînons contenant de 1 à 4 hétéroatomes choisis parmi O, S, N et plus particulièrement : thiophène, furanne, pyrrole ou thiazole, dont les carbones sont éventuellement substitués par un ou plusieurs groupes choisis parmi un radical alkyle ayant de 1 à 6 atomes de carbone linéaire ou ramifié, un radical alkoxy ayant de 1 à 6 atomes de carbone ou un halogène ;
X représente -CO-N(R₃)-X'-, -NH-CO-X'-, -CH=, -CO- ou une liaison, X' représentant -(CH₂)ₙ- avec n entier de 0 à 6 ;
Y représente -Y'-, -CO-NH-Y',-Y'-NH-CO-, -CO-Y'-, -Y'-CO, -N(R₃)-Y'-, -Y'-N(R₃)-, Y'-CH₂-N(R₃)-CO-, -O-Y'-, -Y'-O-, -S-Y'-, -Y'-S-, -Y'-O-Y'-, -Y'-N(R₃)-Y'- ou une liaison, Y' représentant -(CH₂)ₙ- avec n entier de 0 à 6 ;
Het représente un hétérocycle comportant de 1 à 5 hétéroatomes choisis parmi O, N, S pouvant être substitués par un ou plusieurs substituants X'-OR₃, X'-NR₃, X'-S-R₃ et tel que par exemple :
oxétane, pyrrole, pyrrolidine, furanne, tétrahydrofuranne, thiophène, tétrahydrothiophéne, sulfolane, pipérazine, homopipérazine, 4-aminopipéridineimidazole, imidazoline, dihydroimidazole-2-one, dihydroimidazole-2-thione, oxazole, isoxazole, oxazoline, isoxazoline, oxazolidine, oxazolidinone, thiazole, thiazoline, thiazolidine, thiazolidinone, hydantoine, 1,2,4-triazole, 1,3,4-oxadiazole, 1,3,4-thiadiazole, 1,1-dioxyde-1,2,5-thiadiazolidine, 1,2,4-triazole-3-one, tétrazole, tétrahydropyridine, azétidine ;
ou des sels pharmaceutiquement acceptables de ces derniers.

Dans certains cas, les composés de la présente invention peuvent comporter des atomes de carbone asymétriques. Par conséquent, les composés de la présente invention ont deux formes énantiomères possibles, c'est-à-dire les configurations "R" et "S". Les composés de la présente invention incluent les deux formes énantiomères et toutes combinaisons de ces formes, y compris les mélanges racémiques "RS". Dans un souci de simplicité, lorsqu'aucune configuration spécifique n'est indiquée dans les formules de structure, il faut comprendre que les deux formes énantiomères et leurs mélanges sont représentés.

Par alkyle, lorsqu'il n'est pas donné plus de précision, on entend un radical alkyle linéaire ou ramifié comptant de 1 à 6 atomes de carbone. Par alkényle, lorsqu'il n'est pas donné plus de précision, on entend un radical alkyle linéaire ou ramifié comptant de 1 à 6 atomes de carbone et présentant au moins une insaturation (double liaison). Par haloalkyle, on entend un radical alkyle dont au moins l'un des atomes d'hydrogène (et éventuellement tous) est remplacé par un atome halogène.

Par radicaux alkoxy, alkényle ou alkylamino, on entend respectivement les radicaux alkoxy, alkényle ou alkylamino dont le radical alkyle a la signification indiquée précédemment.

Par alkyle linéaire ou ramifié ayant de 1 à 6 atomes de carbone, on entend en particulier les radicaux méthyle, éthyle, propyle, isopropyle, butyle, isobutyle, sec-butyle et tert-butyle, pentyle, néopentyle, isopentyle, hexyle, isohexyle. Enfin, par halogène, on entend les atomes de fluor, de chlore, de brome ou d'iode.

Par sel pharmaceutiquement acceptable, on entend notamment des sels d'addition d'acides inorganiques tels que chlorhydrate, bromhydrate, iodhydrate, sulfate, phosphate, diphosphate et nitrate ou d'acides organiques tels que acétate, maléate, fumarate, tartrate, succinate, citrate, lactate, méthanesulfonate, p-toluènesulfonate, pamoate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Salt selection for basic drugs", *Int. J. Pharm.* (1986), **33,** 201-217.

Parmi les inhibiteurs de phospholipases A2, on peut définir les composés de type acides acylpyrroldicarboxyliques, acides acylindoldicarboxyliques et de dérivés de pyrrolidine. Les inhibiteurs de phospholipases A2 de type acides acylpyrroldicarboxyliques, acylindoldicarboxyliques et de dérivés de pyrrolidine peuvent être des composés tels que décrits dans les demandes EP 97934481, WO 98/05637, EP 969225076 et WO 98/33797. Les inhibiteurs de phospholipases A2 peuvent également être les composés tels que définis dans les demandes WO 99/43672, WO 99/43654, WO 99/43651, WO 99/15493 et WO 98/37069. Les inhibiteurs de phospholipases A2 peuvent notamment être aussi des dérivés d'arylsulfonamides tels que décrits dans la demande WO 98/25893, des dérivés de 1H-indole-3-glyoxylamide tels ceux décrits dans la demande WO 99/57100, des dérivés d'indoles tels ceux décrits dans la demande WO 00/07591 ou des dérivés de 2-phénylpyrimidines tels ceux décrits dans la demande WO 00/27824.

Selon l'invention, les inhibiteurs de phospholipases A2 seront de préférence :
- les composés de formule générale I_{B} ou I'_{B} 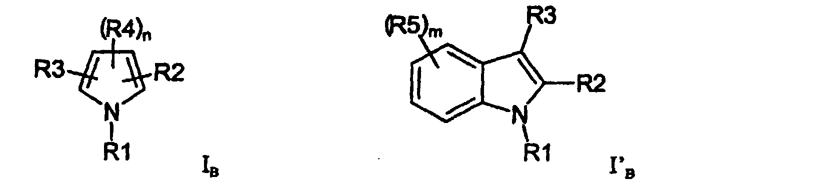
dans lesquelles
R1 représente un groupe -Y1-Ar-Y2-Y3 dans lequel Y1 et Y2 représentent indépendamment un radical (C₁-C₁₂)-alkyle, (C₂-C₁₂)-alkényle, (C₁-C₁₂)-alkoxy ou (C₁-C₁₂)-alkényloxy, les radicaux Y1 et Y2 pouvant le cas échéant être interrompus par un ou plusieurs atomes d'oxygène, Ar est un groupe aryle éventuellement substitué de 1 à 3 fois par des substituants choisis indépendamment parmi les groupes R6, R7 et R8, et Y3 représente un radical -COOR17, -CONR17R17, -CONHCOR19, -CONHS(O)₂R19, -CONHNHS(O)₂R19 ou Tz ;
R2 représente -COOR17, -Y4-COOR17, -CONR17R17, -Y4-CONR17R17, -CONHCOR19, -Y4-CONHCOR19, -CONHS(O)₂R19, -Y4-CONHS(O)₂R19, -CONHNHS(O)₂R19, -Y4-CONHNHS(O)₂R19, Tz ou -Y4-Tz, Y4 représentant un radical (C₁-C₈)-alkyle, (C₂-C₈)-alkényle, le radical Y4 pouvant le cas échéant être interrompu par un ou plusieurs atomes d'oxygène ;
R3 représente un radical -CO-R9 dans lequel R9 représente un radical -Y5, aryle ou -Y5-aryle, Y5 représentant un radical (C₁-C₁₉)-alkyle, (C₂-C₁₉)-alkényle ou (C₂-C₁₉)-alkynyle, le radical Y5 pouvant le cas échéant être interrompu par un ou plusieurs atomes d'oxygène et le radical aryle de R9 étant éventuellement substitué de 1 à 3 fois par des substituants choisis indépendamment parmi les groupes R10, R11 et R12;
chaque radical R4 représente indépendamment un atome d'hydrogène, un atome halogène, -CF₃, -Y6, aryle ou -Y6-aryle, Y6 représentant un radical (C₁-C₈)-alkyle, (C₂-C₈)-alkényle ou (C₂-C₈)-alkynyle, le radical Y5 pouvant le cas échéant être interrompu par un ou plusieurs atomes d'oxygène et le radical aryle de R4 étant éventuellement substitué de 1 à 3 fois par des substituants choisis indépendamment parmi les groupes R13, R14 et R15 et deux radicaux alkyle Y6 voisins pouvant former ensemble avec les atomes de carbone auxquels ils sont attachés un cycle de 5 à 8 chaînons qui, peut lui-même le cas échéant être substitué par un ou deux radicaux (C₁-C₄)-alkyle ;
n représente le chiffre 2 et m le chiffre 4 ;
Tz est un radical 1H- ou 2H-tétrazol-5-yle ;
les radicaux R6, R7, R8, R10, R11, R12, R13, R14, R15 et R16 sont choisis indépendamment parmi le groupe composé des radicaux (C₁-C₂₀)-alkyle, (C₂-C₂₀)-alkényle ou (C₂-C₂₀)-alkynyle éventuellement interrompus par un ou plusieurs atomes d'oxygène, un atome halogène, les groupes -CF₃, -CN, -NO₂ ou -COCH₂OH et un radical perhalo(C₁-C₆)-alkényle, -OR17, -SR17, -COOR17, -COR18, -NHCOR17, -NR17R17, -NHS(O)₂R17, -SOR17, -S(O)₂R17, -CONR17R17, -S(O)₂NR17R17, -OOCR18, -OOCNR17RI7, -OOCOR17, -(CH₂),OR23, -(CH₂)ᵣSR23, -(CH₂)ᵣNHR23 ou -(CH₂)ₛR20 ;
R17 représente, indépendamment à chaque fois qu'il intervient, un radical -(CH₂)ₜR20 ou l'un des radicaux (C₁-C₂₀)-alkyle, (C₂-C₂₀)-alkényle ou (C₂-C₂₀)-alkynyle éventuellement-interrompus par un ou plusieurs atomes d'oxygène ;
R18 représente, indépendamment à chaque fois qu'il intervient, un radical R17, -CF₃, -(CH₂)ᵤCOOH ou-(CH₂)ᵤCOOR21 ;
R19 représente, indépendamment à chaque fois qu'il intervient, un radical R17 ou -CF₃ ;
R20 représente, indépendamment à chaque fois qu'il intervient, un radical aryle substitué par un ou deux groupes R22 ;
R21 représente, indépendamment à chaque fois qu'il intervient, un radical (C₁-C₆)-alkyle, benzyle ou phényle ;
R22 représente, indépendamment à chaque fois qu'il intervient, un atome d'hydrogène, un atome halogène, un radical (C₁-C₁₂)-alkyle, (C₁-C₁₂)-alkoxy, (C₁-C₁₂)-alkylthio, (C₁-C₁₂)-alkylsulfonyle, (C₁-C₁₂)-alkylcarbonyle, -CF₃, -CN ou -NO₂ ;
R23 représente, indépendamment à chaque fois qu'il intervient, un atome d'hydrogène ou un radical -COR21 ;
r est un entier de 1 à 20 ;
s et t représentent indépendamment des entiers de 0 à 12 ;
et u représente un entier de 0 à 4 ;
- ou les composés de formule générale II_{B} 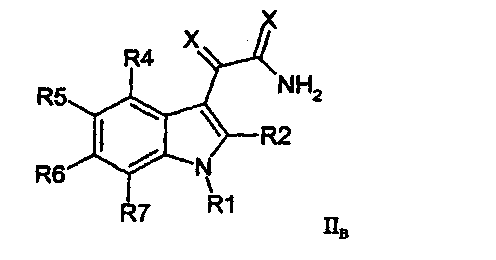
dans laquelle
les deux groupes X représentent des atomes d'oxygène ;
R1 est choisi parmi le groupe constitué par les radicaux 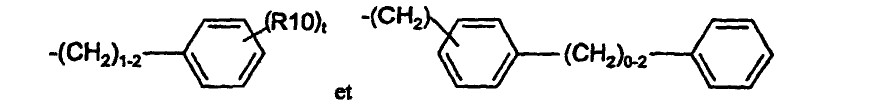 dans lesquels R10 est choisi indépendamment parmi un atome halogène et un radical (C₁-C₁₀)-alkyle, (C₁-C₁₀)-alkoxy, (C₁-C₁₀)-alkylthio et (C₁-C₁₀)-haloalkyle et t est un entier de 0 à 5 ;
R2 est choisi parmi un atome halogène et un radical cyclopropyle, méthyle, éthyle ou propyle ;
R4 et R5 sont choisis indépendamment parmi un atome d'hydrogène, un substituent n'interférant pas ou le groupe (acide) -Lₐ-(groupe acide), étant entendu que -Lₐ- pour R4 est choisi parmi -O-CH₂-, -S-CH₂-, -NH-CH₂-, -CH₂-CH₂-, -O-CH(CH₃)- et -O-CH(CH₂-CH₂-Ph)-, Ph étant un radical phényle et que -Lₐ- pour R5 est choisi parmi les radicaux 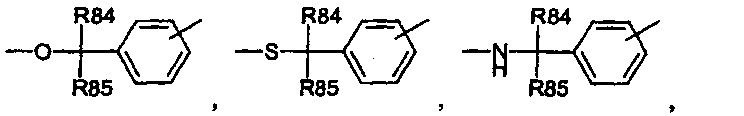 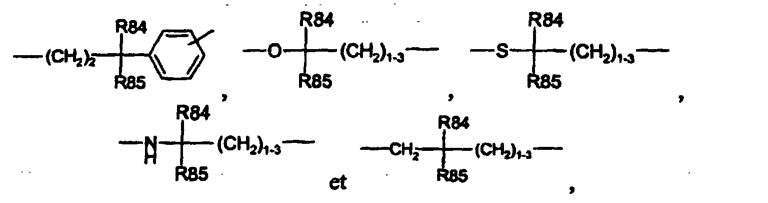 dans lesquels R84 et R85 sont choisis indépendamment parmi un atome d'hydrogène, un atome halogène et un radical (C₁-C₁₀)-alkyle, aryle, (C₁-C₁₀)-alkaryle, (C₁-C₁₀)-aralkyle, carboxy ou carbalkoxy,
étant entendu également que l'un au moins de R4 et R5 doit être le groupe -Lₐ-(groupe acide) et que le (groupe acide) de R4 et R5 est choisi parmi -CO₂H, -SO₃H et -P(O)(OH)₂ ;
R6 et R7 sont choisis indépendamment parmi un atome d'hydrogène et un substituant n'interférant pas ;
les substituants n'interférant pas étant choisis indépendamment parmi le groupe composé des radicaux (C₁-C₆)-alkyle, (C₂-C₆)-alkényle, (C₂-C₆)-alkynyle, (C₇-C₁₈)-aralkyle, (C₇-C₁₈)-alkaryle, (C₃-C₈)-cycloalkyle, (C₃-C₈)-cycloalkényle, phényle, toluyle, xylényle, phénylméthyle, (C₁-C₆)-alkoxy, (C₂-C₆)-alkényloxy, (C₂-C₆)-alkynyloxy, (C₂-C₁₂)-alkoxyalkyl, (C₂-C₁₂)-alkoxyalkyloxy, (C₂-C₁₂)-alkylcarbonylamino, (C₂-C₁₂)-alkoxyamino, (C₂-C₁₂)-alkoxyaminocarbonyle, (C₂-C₁₂)-alkylamino, (C₁-C₆)-alkylthio, (C₂-C₁₂)-alkylthiocarbonyle, (C₁-C₆)-alkylsulfinyle, (C₁-C₆)-alkylsulfonyle, (C₂-C₆)-haloalkoxy, (C₂-C₆)-haloalkylsulfonyle, (C₂-C₆)-haloalkyle, (C₁-C₆)-hydroxyalkyle, -C(O)O((C₁-C₆)-alkyle), -(CH₂)ₙ-O-((C₁-C₆)-alkyle), benzyloxy, phénoxy, phénylthio, -CHO, amino, amidino, bromo, carbamyle, carboxyle, carbalkoxy, -(CH₂)ₙ-CO₂H, chloro, cyano, cyanoguanidinyle, fluoro, guanidino, hydrazide, hydrazino, hydrazido, hydroxy, hydroxyamino, iodo, nitro, phosphono, -SO₃H, thioacétal, thiocarbonyle et (C₁-C₆)-alkylcarbonyle, n étant un entier de 1 à 8 ;
- ou les composés de formule générale III_{B} 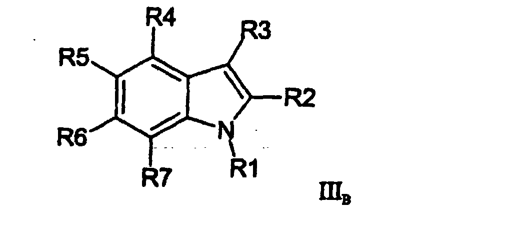
dans laquelle
R1 est choisi parmi les groupes (a), (b) et (c) ;
(a) représente un radical (C₇-C₂₀)-alkyle, (C₇-C₂₀)-haloalkyle, (C₇-C₂₀)-alkényle ou (C₇-C₂₀)-alkynyle, un cycle carboné de 5 à 14 chaînons saturé ou insaturé éventuellement substitué par un ou des substituants choisis parmi les substituants n'interférant pas, ou encore (a) représente un hétérocycle de 5 à 14 chaînons contenant de 1 à 3 hétéroatomes, ledit hétérocycle étant saturé ou insaturé et éventuellement substitué par un ou des substituants choisis parmi les substituants n'interférant pas ;
(b) est l'un des radicaux (a) substitué par un ou plusieurs substituants choisis parmi les substituants n'interférant pas ;
(c) représente le groupe -(L₁)-R11 dans lequel -(L₁)- est un groupe choisi parmi les groupes représentés par les formules 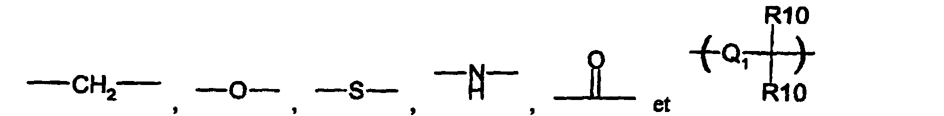 dans lesquelles Q, représente une liaison ou l'un des groupes -CH₂-, -O-, -S-, -NH- et -CO- et chacun des groupes R10 représente indépendamment un atome d'hydrogène ou un radical (C₁-C₈)-alkyle, (C₁-C₈)-haloalkyle ou (C₁-C₈)-alkoxy, et R11 est un groupe choisi parmi les groupes (a) et (b) ;
R2 est un atome d'hydrogène, un atome halogène, ou un radical (C₁-C₄)-alkyle, (C₂-C₄)-alkényle, -O-(C₁-C₃)-alkyle, -S-(C₁-C₃)-alkyle, -(C₁-C₄)-cycloalkyle, -CF₃, -NO₂, -CN ou -SO₃ ;
R3 représente -(L₃)-Z dans lequel -(L₃)- est un groupe de liaison divalent choisi parmi une liaison et les groupes -CH₂-, -O-, -S-, -NH- et -CO- ;
et Z est choisi parmi les groupes acétamide, thioacétamide, glyoxylamide, thioglioxylamide, hydrazide ou thiohydrazide représentés par les formules générales 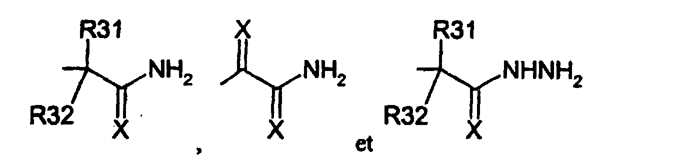 dans lesquelles R31 et R32 sont choisis indépendamment parmi un atome d'hydrogène et un radical (C₁-C₈)-alkyle, (C₁-C₈)-haloalkyle ou (C₃-C₄)-cycloalkyle et X représente, indépendamment à chaque fois qu'il intervient, un atome d'oxygène ou de soufre ;
R4 et R5 sont choisis indépendamment parmi un atome d'hydrogène, un substituant n'interférant pas ou le groupe -(Lₐ)-(groupe acylsulfonamide) dans lequel -(Lₐ)- pour R4 est un groupe représenté par la formule 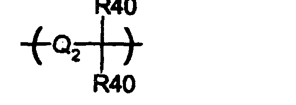 dans laquelle Q₂ est choisi parmi -CH₂-, -O-, -NH-, -C(O)- et -S-, et chacun des groupes R40 est choisi indépendamment parmi un atome d'hydrogène, un atome halogène et les radicaux (C₁-C₈)-alkyle, aryle, (C₁-C₈)-alkaryle, (C₁-C₈)-alkoxy et aralkyle, -(Lₐ)- pour R5 est choisi parmi les groupes représentés par les formules 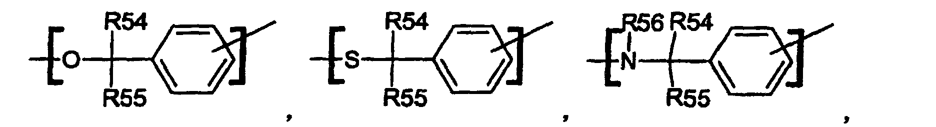 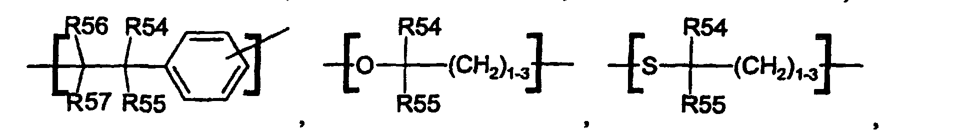 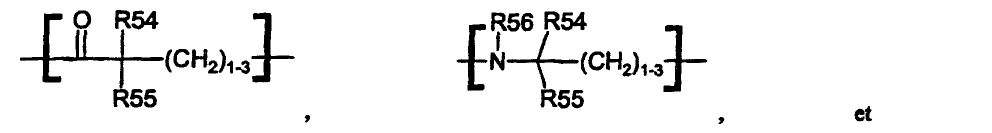 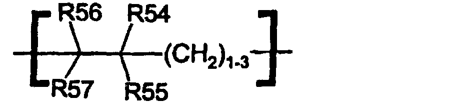 dans lesquelles R54, R55, R56 et R57 sont indépendamment choisis parmi le groupe composé d'un atome d'hydrogène, d'un atome halogène et des radicaux (C₁-C₈)-alkyle, (C₁-C₈)-alkoxy, (C₁-C₈)-haloalkyle et aryle,
le groupe (acylsulfonamide) étant un groupe de formule -CO-NH-SO₂-R81 dans lequel R81 est choisi parmi le groupe composé des radicaux -CF₃, (C₁-C₈)-alkyle, (C₁-C₈)-alkoxy, (C₁-C₈)-alkylthio, (C₁-C₈)-alkylamino, (C₁-C₈)-haloalkyle, (C₁-C₁₄)-aralkyle, (C₁-C₁₄)-alkylaryle, aryle, thioaryle, (C₃-C₁₄)-cycloalkyle et un hétérocycle de 3 à 14 chaînons, ledit hétérocycle comptant de 1 à 3 hétéroaztomes et étant saturé ou insaturé,
étant par ailleurs entendu que l'un au moins de R4 et R5 est un groupe -(Lₐ)-(groupe acylsulfonamide) ;
R6 et R7 représentent indépendamment un atome d'hydrogène, un substituant n'interférant pas, un cycle carboné de 5 à 14 chaînons saturé ou insaturé éventuellement substitué par un ou des substituants choisis panni les substituants n'interférant pas ou un hétérocycle de 5 à 14 chaînons contenant de 1 à 3 hétéroatomes, ledit hétérocycle étant saturé ou insaturé et éventuellement substitué par un ou des substituants choisis parmi les substituants n'interférant pas ;
les substituants n'interférant pas étant choisis indépendamment parmi le groupe composé des radicaux (C₁-C₈)-alkyle, (C₂-C₈)-alkényle, (C₂-C₈)-alkynyle, (C₇-C₁₂)-aralkyle, (C₇-C₁₂)-alkaryle, (C₃-C₈)-cycloalkyle, (C₃-C₈)-cycloalkényle, phényle, toluyle, xylényle, biphényle, (C₁-C₈)-alkoxy, (C₂-C₈)-alkényloxy, (C₂-C₈)-alkynyloxy, (C₂-C₁₂)-alkoxyalkyl, (C₂-C₁₂)-alkoxyalkyloxy, (C₂-C₁₂)-alkylcarbonyle, (C₂-C₁₂)-alkylcarbonylamino, (C₂-C₁₂)-alkoxyamino, (C₂-C₁₂)-alkoxyaminocarbonyle, (C₁-C₁₂)-alkylamino, (C₁-C₆)-alkylthio, (C₂-C₁₂)-alkylthiocarbonyle, (C₁-C₈)-alkylsulfmyle, (C₁-C₈)-alkylsulfonyle, (C₂-C₈)-haloalkoxy, (C₁-C₈)-haloalkylsulfonyle, (C₂-C₈)-haloalkyle, (C₁-C₈)-hydroxyalkyle, -C(O)O((C₁-C₈)-alkyle), -(CH₂)ₙ-O-((C₁-C₈)-alkyle), benzyloxy, phénoxy, phénylthio, -CONHSO₂R, -CHO, amino, amidino, bromo, carbamyle, carboxyle, carbalkoxy, -(CH₂)ₙ-CO₂H, chloro, cyano, cyanoguanidinyle, fluoro, guanidino, hydrazide, hydrazino, hydrazido, hydroxy, hydroxyamino, iodo, nitro, phosphono, -SO₃H, thioacétal, thiocarbonyle, n étant un entier de 1 à 8 et R un radical (C₁-C₈)-alkyle ;
- ou les composés de formule générale IV_{B} 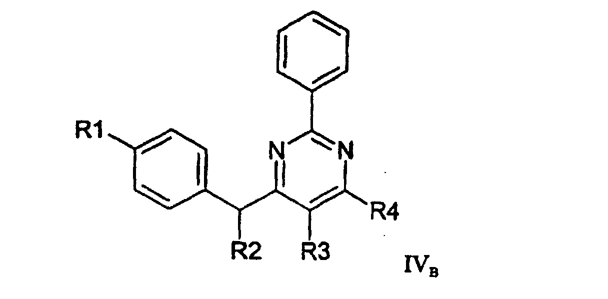
dans laquelle
R1 est choisi parmi le groupe composé d'un atome halogène et des radicaux (C₁-C₆)-alkyle ou (C₁-C₆)-alkoxy ;
R2 est choisi parmi le groupe composé d'un des radicaux phényle ou benzyle éventuellement substitués sur le cycle aromatique par un atome halogène ou un radical (C₁-C₆)-alkyle, (C₁-C₆)-alkoxy, amino, alkylamino, dialkylamino, carboxyle, carbamoyle, (C₁-C₈)-acyl, sulfonyle, thiol ou alkylthio, et d'un radical (C₃-C₇)-cycloalkyle, ledit radical (C₃-C₇)-cycloalkyle étant éventuellement condensé avec un cycle benzénique ou un hétérocycle et étant éventuellement substitué par un atome halogène ou un radical (C₁-C₆)-alkyle ;
R3 est choisi parmi le groupe composé d'un atome halogène et d'un radical (C₁-C₆)-alkyle ;
et R4 est choisi parmi le groupe composé de -H, -OH, -N₃ et -NHCOCH₃ ;
- ou encore les sels pharmaceutiquement acceptables des composés de formule générale I_{B}, II_{B}, III_{B} ou IV_{B}.

Selon une autre variante préférée, les inhibiteurs de phospholipases A2 pourront être choisis parmi le groupe constitué par la mépacrine, l'acide aristolochique et leurs analogues, ainsi que les sels pharmaceutiquement acceptables de ces derniers.

L'invention a plus particulièrement pour objet l'usage d'un produit tel que défini ci-dessus, caractérisé en ce que :
- le ou les inhibiteurs de NO synthases sont choisis parmi le groupe constitué par la L-nitro-arginine (LNA), l'ester méthylique de la L-nitro-arginine (LNAME), la L-N-monométhylarginine (LNMMA), l'aminoguanidine, l'agmatine, le 2-amino-1-(méthylamino)benzimidazole, le 5-nitro-indazole, le 6-nitro-indazole, le 7-nitro-indazole, le 1,2-(trifluorométhylphényl)imidazole (TRIM), la 2-amino-4-méthyl-6-(2-aminoéthyl)pyridine, la 2-iminopipéridine, la 2-iminohomopipéridine, la 2-imino-5,6-dihydro-1,3-thiazine, la 2-imino-5,6-dihyoro-1,3-oxazine, le N-phényl-2-thiophènecarboximidamide, la 2-iminotétrahydrogyrimidine, la S-éthylisothiourée, la S-méthyl-L-thiocitrulline, la S-éthyl-L-thiocitrulline, le (S)-N-{4-[4-[(3,4-dihydro-6-hydroxy-2,5,7,8-tétraméthyl-2H-1-benzopyran-2-yl)-carbonyl]-1-pipérazinyl]phényl}-2-thiophènecarboximidamide et la 4-[3,5-bis(1,1-diméthyléthyl)-4-hydroxyphényl]-N-{4-[(imino(2-thiényl)méthyl)amino]phényl}-N-méthyl-2-thiazoleméthanamine, ainsi que les sels pharmaceutiquement acceptables de ces derniers ; et
- le ou les inhibiteurs de phospholipases A2 sont choisis parmi le groupe constitué par la mépacrine et l'acide aristolochique et leurs analogues, ainsi que les sels pharmaceutiquement acceptables de ces derniers.

Encore plus préférentiellement:
- le ou les inhibiteurs de NO synthases sont choisis parmi le groupe constitué par l'aminoguanidine, le 7-nitro-indazole et la 4-[3,5-bis(1,1-diméthyléthyl)-4-hydroxyphényl]-N-{4-[(imino(2-thiényl)méthyl)amino]phényl}-N-méthyl-2-thiazole-méthanamine, ainsi que les sels pharmaceutiquement acceptables des composés précités ; et
- le ou les inhibitenrs de phospholipases A2 sont choisis parmi le groupe constitué par la mépacrine, l'acide aristolochique et leurs analogues, ainsi que les sels pharmaceutiquement acceptables de ces derniers.

Comme déjà indiqué, un médicament préparé selon l'invention pourra être utilisé dans le traitement de pathologies dans lesquelles le monoxyde d'azote et/ou les phospholipases A2 sont impliquées, choisies parmi les troubles cardiovasculaires et cérébrovasculaires, les troubles du système nerveux central ou périphérique, les maladies prolifératives et inflammatoires, les diarrhées, les vomissements, les irradiations radioactives, les radiations solaires, les transplantations d'organes, les maladies autoimmunes et virales, le cancer, et plus généralement toutes les pathologies caractérisées soit par une production excessive de monoxyde d'azote et/ou des phospholipases A2, soit par un dysfonctionnement lié au monoxyde d'azote et/ou aux phospholipases A2.

Selon une variante préférée, le médicament pourra être utilisé dans le traitement de pathologies choisies parmi des maladies prolifératives et inflammatoires telles que l'athérosclérose, l'hypertension pulmonaire, la glomérulonéphrite, l'hypertension portale, le psoriasis, l'arthrose et l'arthrite rhumatoïde, les fibroses, les amyloïdoses et les inflammations du système gastrointestinal ou du système pulmonaire et des voies aériennes.

Selon une variante également préférée, le médicament pourra être utilisé dans le traitement de maladies autoimmunes et virales telles que le lupus, le sida, les infections parasitaires et virales, le diabète, la sclérose en plaques ou les myopathies.

Dans l'utilisation selon l'invention, l'inhibiteur de NO synthases et l'inhibiteur de phospholipases A2 peuvent se présenter à des doses qui peuvent être identiques ou différentes. Les dosages sont choisis en fonction des composés, associés à des diluants ou excipients appropriés.

L'inhibiteur de NO synthases et l'inhibiteur de phospholipases A2 peuvent être administrés de manière simultanée ou séquentielle, par la même voie d'administration ou par des voies différentes, suivant qu'ils se présentent sous forme séparée ou combinée. De préférence, les voies d'administration sont orale, parentérale ou topique.

Une composition pharmaceutique préparée selon l'invention pourra être sous forme d'un solide, par exemple des poudres, des granules, des comprimés, des gélules, des liposomes ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques pourront aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

Les composés inhibiteurs de NO synthases et les composés inhibiteurs des phospholipases A2 sont commerciaux ou peuvent être préparés par les méthodes connus de l'homme de l'art (ou par analogie à ces dernières). En particulier, les composés de formule générale III_{A} sont décrits dans les demandes PCT WO 98/42696 et WO 98/58934 et les composés de formules générales I_{B} et I'_{B}, II_{B}, III_{B} et IV_{B} sont décrits respectivement dans les demandes de brevet PCT WO 98/05637, WO 99/57100, WO 00/07591 et WO 00/27824.

A moins qu'ils ne soient définis d'une autre manière, tous les termes techniques et scientifiques utilisés ici ont la même signification que celle couramment comprise par un spécialiste ordinaire du domaine auquel appartient cette invention.

Les exemples suivants sont présentés pour illustrer les procédures ci-dessus et ne doivent en aucun cas être considérés comme une limite à la portée de l'invention.

### PARTIE EXPÉRIMENTALE :

### Etude pharmacologique des produits de l'invention

L'activité des composés de l'invention a été évaluée in vivo sur un modèle d'oedème à la carragénine sur la patte de rat.

Des rats mâles Sprague Dawley (Charles River) de 170 à 180 g le jour de l'expérience sont laissés en stabulation 5 à 8 jours dans les conditions d'animalerie et sont mis à jeun sur grilles 18h avant et pendant l'expérience. Les groupes sont constitués de 8 animaux. Les produits sont administrés par voie intrapéritonéale (ip, 2ml/kg), 30 minutes avant la Carragénine 1% injectée par voie sous plantaire dans la patte arrière droite des rats. Le volume de la patte correspondant à l'oedème est mesurée à l'aide d'un pléthysmomètre à eau (Ugo Basile, APELEX) avant l'injection de carragénine (t₀) et à 3h après l'injection. Le volume de la patte permet de déterminer les % d'inflammation ((volume de la patte au temps t après carragénine - volume de cette même patte au temps t₀ avant injection des produits/ volume de la patte au temps t₀) x 100). Tout produit capable de diminuer de manière significative l'intensité de l'oedème (% d'inflammation) sera considérer comme actif. Cette efficacité sera statistiquement déterminée par un test de Student.

Dans les exemples ci-après, on désigne par A l'inhibiteur de NO synthases et par B l'inhibiteur de phospholipases A2.

### Exemple 1

Composé AB, combinaison des principes actifs A et B. Composé A : le 7-nitroindazole, inhibiteur de la forme neuronale des NO synthases. Composé B : la mépacrine, inhibiteur de phospholipase A2.

Composé de l'exemple 1 : 4 groupes d'animaux sont constitués:

| | |
|---|---|
| Groupe 1 | traité avec le véhicule + carragénine. |
| Groupe 2 | traité avec A (25 mg/kg) + carragénine. |
| Groupe 3 | traité avec B (30 mg/kg) + carragénine. |
| Groupe 4 | traité avec AB + carragénine. |

| N° du groupe | % d'inflammation |
|---|---|
| 1 | 58,9 ± 4 |
| 2 | 72,5 ± 16,1 NS |
| 3 | 64,5 ± 3,7 NS |
| 4 | 28 ± 6,2 *** |

| | |
|---|---|
| (NS: résultat non significatif; *** : résultat hautement significatif) | |

Les résultats montrent que le 7-nitroindazole (inhibiteur de NO synthase neuronale) et la mépacrine sont inactifs pour protéger l'animal de l'oedème provoqué par la canagénine. Par contre, l'association des deux composés protège les animaux de l'oedème provoqué par la carragénine de manière hautement significative.

### Exemple 2

Composé AB, combinaison des principes actifs A et B sous forme séparée, avec comme composé A : l'aminoguanidine, inhibiteur de la forme inductible des NO synthases ; et comme composé B : la mépacrine, inhibiteur de phospholipases A2.

Composé de l'exemple 2 : 4 groupes d'animaux sont constitués :

| | |
|---|---|
| Groupe 1 | traité avec le véhicule + carragénine. |
| Groupe 2 | traité avec A (400 mg/kg) + carragénine. |
| Groupe 3 | traité avec B (30 mg/kg) + carragénine. |
| Groupe 4 | traité avec AB + carragénine. |

| N° du groupe | % d'inflammation |
|---|---|
| 1 | 54,3 ± 2,5 |
| 2 | 53,1 ± 3,4 NS |
| 3 | 53,9 ± 3,2 NS |
| 4 | 33±5 ** |

| | |
|---|---|
| (NS: résultat non significatif; ** : résultat hautement significatif) | |

Les résultats montrent que l'aminoguanidine (inhibiteur de NO synthase inductible) et la mépacrine sont inactifs pour protéger l'animal de l'oedème provoqué par la carragénine. Par contre, l'association des deux composés protège les animaux de l'oedème provoqué par la carragénine de manière hautement significative.

### Exemple 3

Composé AB, combinaison des principes actifs A et B sous forme séparée, avec comme composé A : l'aminoguanidine, inhibiteur de la forme inductible des NO synthases ; et comme composé B : l'acide aristolochique, inhibiteur de phospholipases A2.

Composé de l'exemple 3 : 4 groupes d'animaux sont constitués:

| | |
|---|---|
| Groupe 1 | traité avec le véhicule + carragénine. |
| Groupe 2 | traité avec A (400 mg/kg) + carragénine. |
| Groupe 3 | traité avec B (30 mg/kg) + carragénine. |
| Groupe 4 | traité avec AB + carragénine. |

| N° du groupe | % d'inflammation |
|---|---|
| 1 | 57,1 ± 3,9 |
| 2 | 60,7 ± 7,5 NS |
| 3 | 68 ± 5,1 NS |
| 4 | 39,1 ± 4,5 ** |

| | |
|---|---|
| (NS: résultat non significatif ; ** : résultat hautement significatif) | |

Les résultats montrent que l'aminoguanidine (inhibiteur de NO synthase inductible) et l'acide aristolochique sont inactifs pour protéger l'animal de l'oedème provoqué par la carragénine. Par contre, l'association des deux composés protège les animaux de l'oedème provoqué par la carragénine de manière hautement significative.

### Exemple 4

Composé AB, combinaison des principes actifs A et B sous forme séparée, avec comme composé A: le chlorhydrate de 4-[3,5-bis(1,1-diméthyléthyl)-4-hydroxyphényl]-N-{4-[(imino(2-thiényl)méthyl)amino]phényl}-N-méthyl-2-thiazoleméthanamine, un inhibiteur mixte de la forme neuronale des NO synthases et de la peroxydation lipidique ; et comme composé B : la mépacrine, inhibiteur de phospholipases A2.

Composé de l'exemple 4 : 4 groupes d'animaux sont constitués:

| | |
|---|---|
| Groupe 1 | traité avec le véhicule + carragénine. |
| Groupe 2 | traité avec A (30 mg/kg) + carragénine. |
| Groupe 3 | traité avec B (30 mg/kg) + carragénine. |
| Groupe 4 | traité avec AB + carragénine. |

| N° du groupe | % d'inflammation |
|---|---|
| 1 | 51,5 ± 2,2 |
| 2 | 52,8 ± 4,0 NS |
| 3 | 51,9 ± 2,7 NS |
| 4 | 37,4 ± 4,6 * |

| | |
|---|---|
| (NS: résultat non significatif; * : résultat significatif) | |

Ici encore, tandis que le chlorhydrate de 4-[3,5-bis(1,1-diméthyléthyl)-4-hydroxyphényl]-N-{4-[(imino(2-thiényl)méthyl)amino]phényl}-N-méthyl-2-thiazole-méthanamine (inhibiteur de NO synthase neuronale et anti-oxydant) et la mépacrine sont inactifs pour protéger l'animal de l'oedème provoqué par la carragénine, l'association des deux composés protège les animaux de l'oedème provoqué par la carragénine de manière significative.

Les résultats expérimentaux des exemples 1, 2, 3 et 4 montrent donc un effet synergique d'action entre les deux types de composés.

### Conclusion

Les résultats montrent que l'administration d'un inhibiteur de NO synthase et d'un inhibiteur de phospholipases A2 inactifs par eux-mêmes, ont un effet anti-inflammatoire synergique lorsqu'ils sont associés.

## Revendications

1. Utilisation d'un produit comprenant au moins une substance inhibitrice de NO synthases en association avec au moins une substance inhibitrice de phospholipases A2, sous forme séparée ou en combinaison, pour préparer un médicament destiné à traiter une pathologie ou un désordre choisi parmi les troubles cardiovasculaires et cérébrovasculaires, les troubles du système nerveux central ou périphérique, les maladies prolifératives et inflammatoires, les diarrhées, les vomissements, les irradiations radioactives, les radiations solaires, les transplantations d'organes, les maladies autoimmunes et virales et le cancer.

2. Utilisation selon la revendication 1, **caractérisée en ce que** la substance inhibitrice de NO synthases est une substance inhibant la forme neuronale des NO synthases.

3. Utilisation selon la revendication 1, **caractérisée en ce que** la substance inhibitrice de NO synthases est une substance inhibant la forme inductible des NO synthases.

4. Utilisation selon l'un des revendications 1 à 3, **caractérisée en ce que** la substance inhibitrice de NO synthases et la substance inhibitrice de phospholipases A2 sont utilisées sous forme séparée.

5. Utilisation selon l'un des revendications 1 à 3, **caractérisée en ce que** la substance inhibitrice de NO synthases et la substance inhibitrice de phospholipases A2 sont sous forme de sel.

6. Utilisation selon la revendication 5, **caractérisée en ce que** le sel est formé à partir d'un dérivé de la substance inhibitrice de NO synthases contenant au moins un groupe basique et d'un dérivé de la substance inhibitrice de phospholipases A2 contenant au moins un groupe acide.

7. Utilisation selon l'une des revendications précédentes, **caractérisée en ce que** :
• le ou les inhibiteurs de NO synthases sont choisis parmi le groupe constitué par :
- un composé de formule générale I_{A} 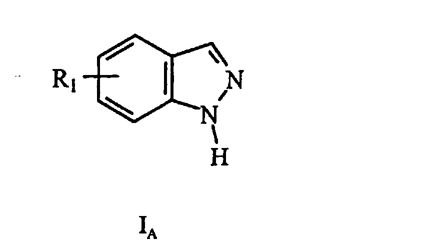
dans laquelle R₁ représente un ou plusieurs substituants choisis parmi l'atome d'hydrogène et les radicaux nitro, halo, alkyle ou alkoxy ;
ou un sel pharmaceutiquement acceptable de ce dernier ;
- un composé de formule générale II_{A} 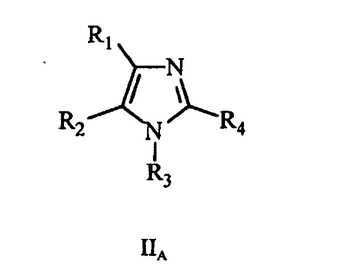
dans laquelle
R₁ et R₂ représentent, indépendamment l'atome d'hydrogène ou le radical halo, hydroxy, amino, alkyle ou alkoxy, ou R₁ et R₂ sont liés ensemble et forment un radical phényle condensé avec le cycle imidazole, ledit radical phényle étant éventuellement substitué par un ou plusieurs substituants choisis parmi les radicaux hydroxy, trifluorométhyle, halo, carboxy, alkyle, alkoxy ou alkényle ;
R₃ représente un atome d'hydrogène ou un radical alkyle, amino, alkylamino ou phényle, le radical phényle étant éventuellement substitué par un ou plusieurs substituants choisis parmi les radicaux hydroxy, trifluorométhyle, halo, carboxy, alkyle, alkoxy inférieur ou alkényle ;
et R₄ représente l'atome d'hydrogène, un radical alkyle, amino ou alkylamino ;
ou un sel pharmaceutiquement acceptable de ce dernier ;
- un composé de formule générale III_{A} 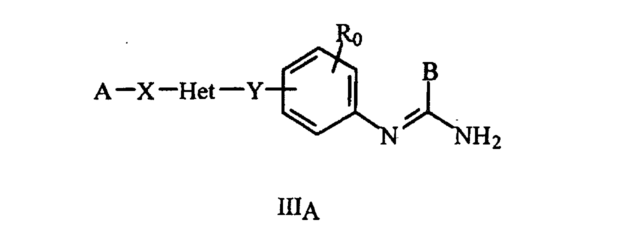
dans laquelle
R₀ représente H ou un radical alkyle ;
A est un radical 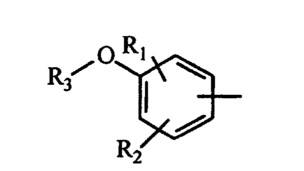 dans lequel R₁ et R₂ représentent, indépendamment, un atome d'hydrogène, un halogène, le groupe OH, un radical alkyle linéaire ou ramifié ayant de 1 à 6 atomes de carbone, un radical alkoxy linéaire ou ramifié ayant de 1 à 6 atomes de carbone,
et R₃ représente un atome d'hydrogène, un radical alkyle linéaire ou ramifié ayant de 1 à 6 atomes de carbone ou un radical -COR₄, R₄ représentant un radical alkyle ayant de 1 à 6 atomes de carbone,
ou un radical 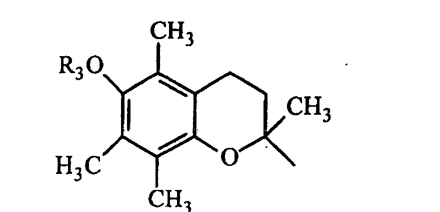 dans lequel R₃ représente un atome d'hydrogène, un radical alkyle linéaire ou ramifié ayant de 1 à 6 atomes de carbone ou un radical -COR₄, R₄ représentant un radical alkyle ayant de 1 à 6 atomes de carbone ;
B représente un radical alkyle linéaire ou ramifié ayant de 1 à 6 atomes de carbone, phényle, pyridinyle ou un hétérocycle à 5 chaînons contenant de 1 à 4 hétéroatomes choisis parmi O, S, N et plus particulièrement : thiophène, furanne, pyrrole ou thiazole, dont les carbones sont éventuellement substitués par un ou plusieurs groupes choisis parmi un radical alkyle ayant de 1 à 6 atomes de carbone linéaire ou ramifié, un radical alkoxy ayant de 1 à 6 atomes de carbone ou un halogène ;
X représente -CO-N(R₃)-X'-, -NH-CO-X'-, -CH=, -CO- ou une liaison, X' représentant -(CH₂)ₙ- avec n entier de 0 à 6 ;
Y représente -Y'-, -CO-NH-Y',-Y'-NH-CO-, -CO-Y'-, -Y'-CO, -N(R₃)-Y'-, -Y'-N(R₃)-, Y'-CH₂-N(R₃)-CO-, -O-Y'-, -Y'-O-, -S-Y'-, -Y'-S-, -Y'-O-Y'-, -Y'-N(R₃)-Y'- ou une liaison, Y' représentant -(CH₂)ₙ- avec n entier de 0 à 6 ;
Het représente un hétérocycle comportant de 1 à 5 hétéroatomes choisis parmi O, N, S pouvant être substitués par un ou plusieurs substituants X'-OR₃, X'-NR₃, X'-S-R₃ et tel que par exemple :
oxétane, pyrrole, pyrrolidine, furanne, tétrahydrofuranne, thiophène, tétrahydrothiophène, sulfolane, pipérazine, homopipérazine, 4-aminopipéridineimidazole, imidazoline, dihydroimidazole-2-one, dihydroimidazole-2-thione, oxazole, isoxazole, oxazoline, isoxazoline, oxazolidine, oxazolidinone, thiazole, thiazoline, thiazolidine, thiazolidinone, hydantoïne, 1,2,4-triazole, 1,3,4-oxadiazole, 1,3,4-thiadiazole, 1,1-dioxyde-1,2,5-thiadiazolidine, 1,2,4-triazole-3-one, tétrazole, tétrahydropyridine, azétidine ;
ou un sel pharmaceutiquement acceptable de ce dernier ;
• le ou les inhibiteurs de phospholipases A2 sont choisis parmi le groupe constitué par la mépacrine, l'acide aristolochique et leurs analogues, ainsi que les sels pharmaceutiquement acceptables de ces derniers.

8. Utilisation selon l'une des revendications 1 à 6, **caractérisé en ce que** le ou les inhibiteurs de phospholipases A2 sont choisis parmi le groupe constitué par :
• les composés de formule générale I_{B} ou I'_{B} 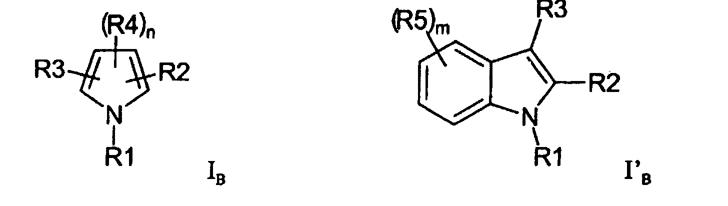
dans lesquelles
R1 représente un groupe -Y1-Ar-Y2-Y3 dans lequel Y1 et Y2 représentent indépendamment un radical (C₁-C₁₂)-alkyle, (C₂-C₁₂)-alkényle, (C₁-C₁₂)-alkoxy ou (C₁-C₁₂)-alkényloxy, les radicaux Y1 et Y2 pouvant le cas échéant être interrompus par un ou plusieurs atomes d'oxygène, Ar est un groupe aryle éventuellement substitué de 1 à 3 fois par des substituants choisis indépendamment parmi les groupes R6, R7 et R8, et Y3 représente un radical -COOR17, -CONR17R17, -CONHCOR19, -CONHS(O)₂R19, -CONHNHS(O)₂R19 ou Tz ;
R2 représente -COOR17, -Y4-COOR17, -CONR17RI7, -Y4-CONR17R17, - CONHCOR19, -Y4-CONHCOR19, -CONHS(O)₂R19, -Y4-CONHS(O)₂R19, - CONHNHS(O)₂R19, -Y4-CONHNHS(O)₂R19, Tz ou -Y4-Tz, Y4 représentant un radical (C₁-C₈)-alkyle, (C₂-C₈)-alkényle, le radical Y4 pouvant le cas échéant être interrompu par un ou plusieurs atomes d'oxygène ;
R3 représente un radical -CO-R9 dans lequel R9 représente un radical -Y5, aryle ou -Y5-aryle, Y5 représentant un radical (C₁-C₁₉)-alkyle, (C₂-C₁₉)-alkényle ou (C₂-C₁₉)-alkynyle, le radical Y5 pouvant le cas échéant être interrompu par un ou plusieurs atomes d'oxygène et le radical aryle de R9 étant éventuellement substitué de 1 à 3 fois par des substituants choisis indépendamment parmi les groupes R10, R11 et R12 ;
chaque radical R4 représente indépendamment un atome d'hydrogène, un atome halogène, -CF₃, -Y6, aryle ou -Y6-aryle, Y6 représentant un radical (C₁-C₈)-alkyle, (C₂-C₈)-alkényle ou (C₂-C₈)-alkynyle, le radical Y5 pouvant le cas échéant être interrompu par un ou plusieurs atomes d'oxygène et le radical aryle de R4 étant éventuellement substitué de 1 à 3 fois par des substituants choisis indépendamment parmi les groupes R13, R14 et R15 et deux radicaux alkyle Y6 voisins pouvant former ensemble avec les atomes de carbone auxquels ils sont attachés un cycle de 5 à 8 chaînons qui peut lui-même le cas échéant être substitué par un ou deux radicaux (C₁-C₄)-alkyle ;
n représente le chiffre 2 et m le chiffre 4 ;
Tz est un radical 1H- ou 2H-tétrazol-5-yle ;
les radicaux R6, R7, R8, R10, R11, R12, R13, R14, R15 et R16 sont choisis indépendamment parmi le groupe composé des radicaux (C₁-C₂₀)-alkyle, (C₂-C₂₀)-alkényle ou (C₂-C₂₀)-alkynyle éventuellement interrompus par un ou plusieurs atomes d'oxygène, un atome halogène, les groupes -CF₃, -CN, -NO₂ ou -COCH₂OH et un radical perhalo(C₁-C₆)-alkényle, -OR17, -SR17, -COOR17, -COR18, -NHCOR17, -NR17R17, -NHS(O)₂R17, -SOR17, -S(O)₂R17, -CONR17R17, -S(O)₂NR17R17, -OOCR18, -OOCNR17R17, -OOCOR17, -(CH₂)ᵣOR23, -(CH₂)ᵣSR23, -(CH₂)ᵣNHR23 ou -(CH₂)ₛR20 ;
R17 représente, indépendamment à chaque fois qu'il intervient, un radical -(CH₂)ᵣR20 ou l'un des radicaux (C₁-C₂₀)-alkyle, (C₂-C₂₀)-alkényle ou (C₂-C₂₀)-alkynyle éventuellement interrompus par un ou plusieurs atomes d'oxygène ; R18 représente, indépendamment à chaque fois qu'il intervient, un radical R17, -CF₃, -(CH₂)ᵤCOOH ou-(CH₂)ᵤCOOR21 ;
R19 représente, indépendamment à chaque fois qu'il intervient, un radical R17 ou -CF₃ ;
R20 représente, indépendamment à chaque fois qu'il intervient, un radical aryle substitué par un ou deux groupes R22 ;
R21 représente, indépendamment à chaque fois qu'il intervient, un radical (C₁-C₆)-alkyle, benzyle ou phényle ;
R22 représente, indépendamment à chaque fois qu'il intervient, un atome d'hydrogène, un atome halogène, un radical (C₁-C₁₂)-alkyle, (C₁-C₁₂)-alkoxy, (C₁-C₁₂)-alkylthio, (C₁-C₁₂)-alkylsulfonyle, (C₁-C₁₂)-alkylcarbonyle, -CF₃, -CN ou -NO₂ ;
R23 représente, indépendamment à chaque fois qu'il intervient, un atome d'hydrogène ou un radical -COR21 ;
r est un entier de 1 à 20 ;
s et t représentent indépendamment des entiers de 0 à 12 ;
et u représente un entier de 0 à 4 ;
• les composés de formule générale II_{B} 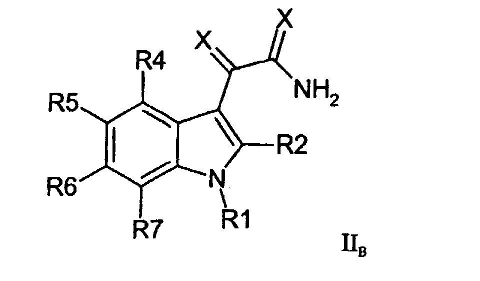
dans laquelle
les deux groupes X représentent des atomes d'oxygène ;
R1 est choisi parmi le groupe constitué par les radicaux 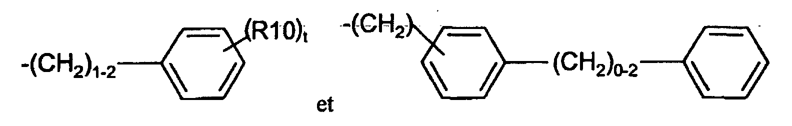 dans lesquels R10 est choisi indépendamment parmi un atome halogène et un radical (C₁-C₁₀)-alkyle, (C₁-C₁₀)-alkoxy, (C₁-C₁₀)-alkylthio et (C₁-C₁₀)-haloalkyle et t est un entier de 0 à 5 ;
R2 est choisi parmi un atome halogène et un radical cyclopropyle, méthyle, éthyle ou propyle ;
R4 et R5 sont choisis indépendamment parmi un atome d'hydrogène, un substituent n'interférant pas ou le groupe (acide) -Lₐ-(groupe acide), étant entendu que -Lₐ- pour R4 est choisi parmi -O-CH₂-, -S-CH₂-, -NH-CH₂-, -CH₂-CH₂-, -O-CH(CH₃)- et -O-CH(CH₂-CH₂-Ph)-, Ph étant un radical phényle et que -Lₐ- pour R5 est choisi parmi les radicaux 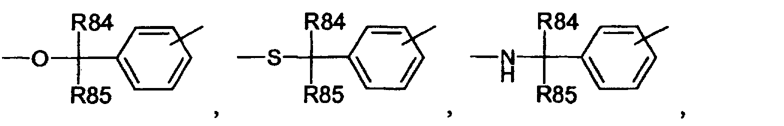 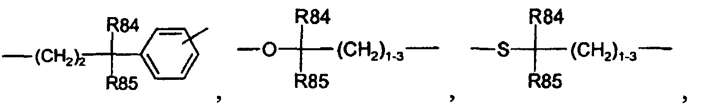 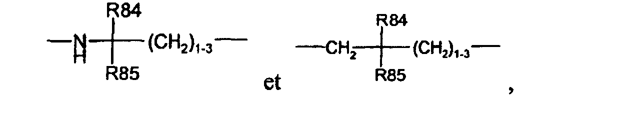 dans lesquels R84 et R85 sont choisis indépendamment parmi un atome d'hydrogène, un atome halogène et un radical (C₁-C₁₀)-alkyle, aryle, (C₁-C₁₀)-alkaryle, (C₁-C₁₀)-aralkyle, carboxy ou carbalkoxy,
étant entendu également que l'un au moins de R4 et R5 doit être le groupe -Lₐ-(groupe acide) et que le (groupe acide) de R4 et R5 est choisi parmi -CO₂H, -SO₃H et -P(O)(OH)₂ ;
R6 et R7 sont choisis indépendamment parmi un atome d'hydrogène et un substituant n'interférant pas ;
les substituants n'interférant pas étant choisis indépendamment parmi le groupe composé des radicaux (C₁-C₆)-alkyle, (C₂-C₆)-alkényle, (C₂-C₆)-alkynyle, (C₇-C₁₈)-aralkyle, (C₇-C₁₈)-alkaryle, (C₃-C₈)-cycloalkyle, (C₃-C₈)-cycloalkényle, phényle, toluyle, xylényle, phénylméthyle, (C₁-C₆)-alkoxy, (C₂-C₆)-alkényloxy, (C₂-C₆)-alkynyloxy, (C₂-C₁₂)-alkoxyalkyl, (C₂-C₁₂)-alkoxyalkyloxy, (C₂-C₁₂)-alkylcarbonylamino, (C₂-C₁₂)-alkoxyamino, (C₂-C₁₂)-alkoxyaminocarbonyle, (C₂-C₁₂)-alkylamino, (C₁-C₆)-alkylthio, (C₂-C₁₂)-alkylthiocarbonyle, (C₁-C₆)-alkylsulfinyle, (C₁-C₆)-alkylsulfonyle, (C₂-C₆)-haloalkoxy, (C₂-C₆)-haloalkylsulfonyle, (C₂-C₆)-haloalkyle, (C₁-C₆)-hydroxyalkyle, -C(O)O((C₁-C₆)-alkyle), -(CH₂)ₙ-O-((C₁-C₆)-alkyle), benzyloxy, phénoxy, phénylthio, -CHO, amino, amidino, bromo, carbamyle, carboxyle, carbalkoxy, -(CH₂)ₙ-CO₂H, chloro, cyano, cyanoguanidinyle, fluoro, guanidino, hydrazide, hydrazino, hydrazido, hydroxy, hydroxyamino, iodo, nitro, phosphono, -SO₃H, thioacétal, thiocarbonyle et (C₁-C₆)-alkylcarbonyle, n étant un entier de 1 à 8 ;
• les composés de formule générale III_{B} 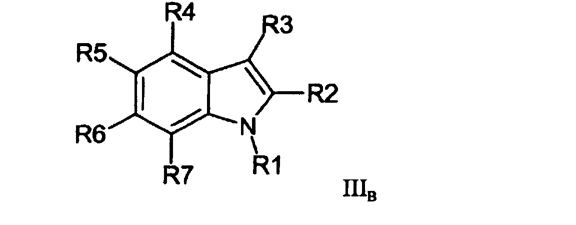
dans laquelle
R1 est choisi parmi les groupes (a), (b) et (c) ;
(a) représente un radical (C₇-C₂₀)-alkyle, (C₇-C₂₀)-haloalkyle, (C₇-C₂₀)-alkényle ou (C₇-C₂₀)-alkynyle, un cycle carboné de 5 à 14 chaînons saturé ou insaturé éventuellement substitué par un ou des substituants choisis parmi les substituants n'interférant pas, ou encore (a) représente un hétérocycle de 5 à 14 chaînons contenant de 1 à 3 hétéroatomes, ledit hétérocycle étant saturé ou insaturé et éventuellement substitué par un ou des substituants choisis parmi les substituants n'interférant pas ;
(b) est l'un des radicaux (a) substitué par un ou plusieurs substituants choisis parmi les substituants n'interférant pas ;
(c) représente le groupe -(L₁)-R11 dans lequel -(L₁)- est un groupe choisi parmi les groupes représentés par les formules 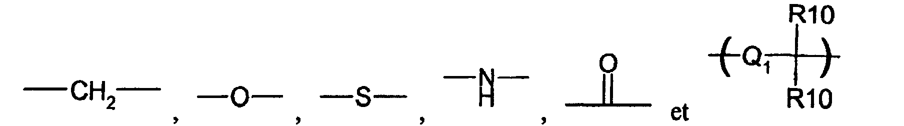
dans lesquelles Q, représente une liaison ou l'un des groupes -CH₂-, -O-, -S-, -NH- et -CO- et chacun des groupes R10 représente indépendamment un atome d'hydrogène ou un radical (C₁-C₈)-alkyle, (C₁-C₈)-haloalkyle ou (C₁-C₈)-alkoxy, et R11 est un groupe choisi parmi les groupes (a) et (b) ;
R2 est un atome d'hydrogène, un atome halogène, ou un radical (C₁-C₄)-alkyle, (C₂-C₄)-alkényle, -O-(C₁-C₃)-alkyle, -S-(C₁-C₃)-alkyle, -(C₁-C₄)-cycloalkyle, -CF₃, -NO₂, -CN ou -SO₃ ;
R3 représente -(L₃)-Z dans lequel -(L₃)- est un groupe de liaison divalent choisi parmi une liaison et les groupes -CH₂-, -O-, -S-, -NH- et -CO- ;
et Z est choisi parmi les groupes acétamide, thioacétamide, glyoxylamide, thioglioxylamide, hydrazide ou thiohydrazide représentés par les formules générales 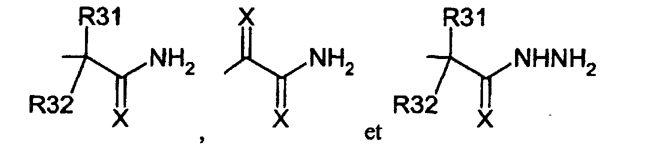 dans lesquelles R31 et R32 sont choisis indépendamment parmi un atome d'hydrogène et un radical (C₁-C₈)-alkyle, (C₁-C₈)-haloalkyle ou (C₃-C₄)-cycloalkyle et X représente, indépendamment à chaque fois qu'il intervient, un atome d'oxygène ou de soufre ;
R4 et R5 sont choisis indépendamment parmi un atome d'hydrogène, un substituant n'interférant pas ou le groupe -(Lₐ)-(groupe acylsulfonamide) dans lequel -(Lₐ)- pour R4 est un groupe représenté par la formule 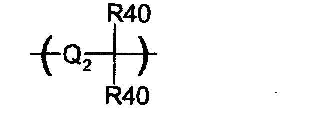 dans laquelle Q₂ est choisi parmi -CH₂-, -O-, -NH-, -C(O)- et -S-, et chacun des groupes R40 est choisi indépendamment parmi un atome d'hydrogène, un atome halogène et les radicaux (C₁-C₈)-alkyle, aryle, (C₁-C₈)-alkaryle, (C₁-C₈)-alkoxy et aralkyle, -(Lₐ)- pour R5 est choisi parmi les groupes représentés par les formules 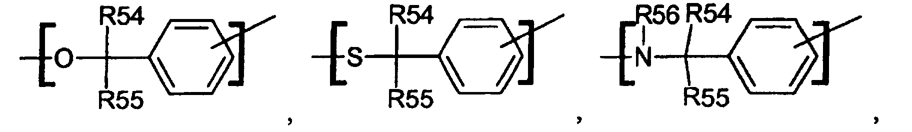 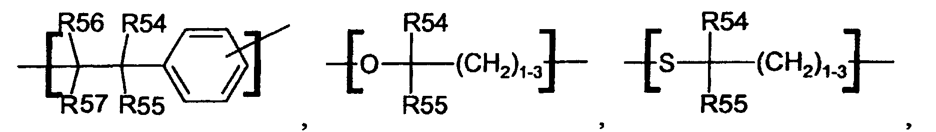 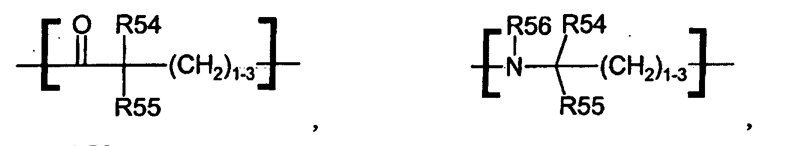 et 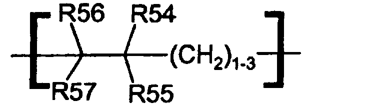 dans lesquelles R54, R55, R56 et R57 sont indépendamment choisis parmi le groupe composé d'un atome d'hydrogène, d'un atome halogène et des radicaux (C₁-C₈)-alkyle, (C₁-C₈)-alkoxy, (C₁-C₈)-haloalkyle et aryle,
le groupe (acylsulfonamide) étant un groupe de formule -CO-NH-SO₂-R81 dans lequel R81 est choisi parmi le groupe composé des radicaux -CF₃, (C₁-C₈)-alkyle, (C₁-C₈)-alkoxy, (C₁-C₈)-alkylthio, (C₁-C₈)-alkylamino, (C₁-C₈)-haloalkyle, (C₁-C₁₄)-aralkyle, (C₁-C₁₄)-alkylaryle, aryle, thioaryle, (C₃-C₁₄)-cycloalkyle et un hétérocycle de 3 à 14 chaînons, ledit hétérocycle comptant de 1 à 3 hétéroaztomes et étant saturé ou insaturé,
étant par ailleurs entendu que l'un au moins de R4 et R5 est un groupe - (LJ-(groupe acylsulfonamide) ;
R6 et R7 représentent indépendamment un atome d'hydrogène, un substituant n'interférant pas, un cycle carboné de 5 à 14 chaînons saturé ou insaturé éventuellement substitué par un ou des substituants choisis parmi les substituants n'interférant pas ou un hétérocycle de 5 à 14 chaînons contenant de 1 à 3 hétéroatomes, ledit hétérocycle étant saturé ou insaturé et éventuellement substitué par un ou des substituants choisis parmi les substituants n'interférant pas ;
les substituants n'interférant pas étant choisis indépendamment parmi le groupe composé des radicaux (C₁-C₈)-alkyle, (C₂-C₈)-alkényle, (C₂-C₈)-alkynyle, (C₇-C₁₂)-aralkyle, (C₇-C₁₂)-alkaryle, (C₃-C₈)-cycloalkyle, (C₃-C₈)-cycloalkényle, phényle, toluyle, xylényle, biphényle, (C₁-C₈)-alkoxy, (C₂-C₈)-alkényloxy, (C₂-C₈)-alkynyloxy, (C₂-C₁₂)-alkoxyalkyl, (C₂-C₁₂)-alkoxyalkyloxy, (C₂-C₁₂)-alkylcarbonyle, (C₂-C₁₂)-alkylcarbonylamino, (C₂-C₁₂)-alkoxyamino, (C₂-C₁₂)-alkoxyaminocarbonyle, (C₁-C₁₂)-alkylamino, (C₁-C₆)-alkylthio, (C₂-C₁₂)-alkylthiocarbonyle, (C₁-C₈)-alkylsulfinyle, (C₁-C₈)-alkylsulfonyle, (C₂-C₈)-haloalkoxy, (C₁-C₈)-haloalkylsulfonyle, (C₂-C₈)-haloalkyle, (C₁-C₈)-hydroxyalkyle, -C(O)O((C₁-C₈)-alkyle), -(CH₂)ₙ-O-((C₁-C₈)-alkyle), benzyloxy, phénoxy, phénylthio, -CONHSO₂R, -CHO, amino, amidino, bromo, carbamyle, carboxyle, carbalkoxy, -(CH₂)ₙ-CO₂H, chloro, cyano, cyanoguanidinyle, fluoro, guanidino, hydrazide, hydrazino, hydrazido, hydroxy, hydroxyamino, iodo, nitro, phosphono, -SO₃H, thioacétal, thiocarbonyle, n étant un entier de 1 à 8 et R un radical (C₁-C₈)-alkyle ;
• les composés de formule générale IV_{B} 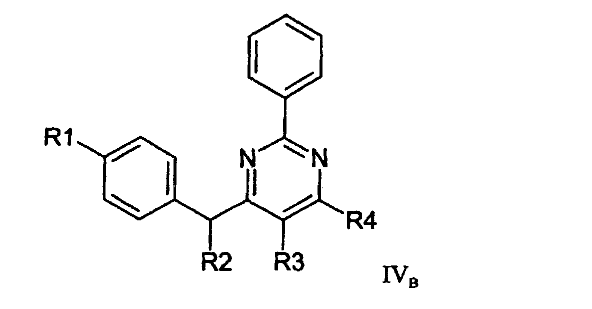
dans laquelle
R1 est choisi parmi le groupe composé d'un atome halogène et des radicaux (C₁-C₆)-alkyle ou (C₁-C₆)-alkoxy ;
R2 est choisi parmi le groupe composé d'un des radicaux phényle ou benzyle éventuellement substitués sur le cycle aromatique par un atome halogène ou un radical (C₁-C₆)-alkyle, (C₁-C₆)-alkoxy, amino, alkylamino, dialkylamino, carboxyle, carbamoyle, (C₁-C₈)-acyl, sulfonyle, thiol ou alkylthio, et d'un radical (C₃-C₇)-cycloalkyle, ledit radical (C₃-C₇)-cycloalkyle étant éventuellement condensé avec un cycle benzénique ou un hétérocycle et étant éventuellement substitué par un atome halogène ou un radical (C₁-C₆)-alkyle ;
R3 est choisi parmi le groupe composé d'un atome halogène et d'un radical (C₁-C₆)-alkyle ;
et R4 est choisi parmi le groupe composé de -H, -OH, -N₃ et -NHCOCH₃ ;
• et les sels pharmaceutiquement acceptables des composés de formule générale I_{B}, II_{B}, III_{B} ou IV_{B}.

9. Utilisation selon la revendication 1, **caractérisée en ce que** :
• le ou les inhibiteurs de NO synthases sont choisis parmi le groupe constitué par la L-nitro-arginine (LNA), l'ester méthylique de la L-nitro-arginine (LNAME), la L-N-monométhylarginine (LNMMA), l'aminoguanidine, l'agmatine, le 2-amino-1-(méthylamino)benzimidazole, le 5-nitro-indazole, le 6-nitro-indazole, le 7-nitro-indazole, le 1,2-(trifluorométhylphényl)imidazole (TRIM), la 2-amino-4-méthyl-6-(2-aminoéthyl)pyridine, la 2-iminopipéridine, la 2-iminohomopipéridine, la 2-imino-5,6-dihydro-1,3-thiazine, la 2-imino-5,6-dihydro-1,3-oxazine, le N-phényl-2-thiophènecarboximidamide, la 2-iminotétrahydropyrimidine, la S-éthylisothiourée, la S-méthyl-L-thiocitrulline, la S-éthyl-L-thiocitrulline, le (S)-N- {4-[4-[(3,4-dihydro-6-hydroxy-2,5,7,8-tétraméthyl-2H-1-benzopyran-2-yl)-carbonyl]-1-pipérazinyl]phényl}-2-thiophènecarboximidamide et la 4-[3,5-bis(1,1-diméthyléthyl)-4-hydroxyphényl]-N-{4-[(imino(2-thiényl)méthyl)amino]phényl}-N-méthyl-2-thiazoleméthanamine, ainsi que les sels pharmaceutiquement acceptables de ces derniers ; et
• le ou les inhibiteurs de phospholipases A2 sont choisis parmi le groupe constitué par la mépacrine et l'acide aristolochique et leurs analogues, ainsi que les sels pharmaceutiquement acceptables de ces derniers.

10. Utilisation selon la revendication 9, **caractérisée en ce que** :
• le ou les inhibiteurs de NO synthases sont choisis parmi le groupe constitué par l'aminoguanidine, le 7-nitro-indazole et la 4-[3,5-bis(1,1-diméthyléthyl)-4-hydroxyphényl]-N-{4-[(imino(2-thiényl)méthyl)amino]phényl}-N-méthyl-2-thiazole-méthanamine, ainsi que les sels pharmaceutiquement acceptables des composés précités ; et
• le ou les inhibiteurs de phospholipases A2 sont choisis parmi le groupe constitué par la mépacrine et l'acide aristolochique et leurs analogues, ainsi que les sels pharmaceutiquement acceptables de ces derniers.

11. Utilisation selon l'une des revendications 1 à 10, **caractérisée en ce que** les pathologies ou les désordres sont choisis parmi les maladies prolifératives et inflammatoires telles que l'athérosclérose, l'hypertension pulmonaire, la glomérulonéphrite, l'hypertension portale, le psoriasis, l'arthrose et l'arthrite rhumatoïde, les fibroses, les amyloïdoses et les inflammations du système gastrointestinal ou du système pulmonaire et des voies aériennes.

12. Utilisation selon l'une des revendications 1 à 10, **caractérisée en ce que** les pathologies ou les désordres sont choisis parmi les maladies autoimmunes et virales telles que le lupus, le sida, les infections parasitaires et virales, le diabète, la sclérose en plaques ou les myopathies.

## Claims

1. Use of a product comprising at least one NO synthase inhibiting substance in association with at least one phospholipase A2 inhibiting substance, in separate form or in combination, for preparing a medicament intended to treat a pathology or disorder chosen from the cardiovascular and cerebrovascular disorders, disorders of the central or peripheral nervous system, proliferative and inflammatory diseases, diarrheas, vomiting, radioactive irradiation, solar radiation, organ transplants, autoimmune and viral diseases and cancer.

2. Use according to claim 1, **characterized in that** the NO synthase inhibiting substance is a substance inhibiting the neuronal form of NO synthases.

3. Use according to claim 1, **characterized in that** the NO synthase inhibiting substance is a substance inhibiting the inducible form of NO synthases.

4. Use according to one of claims 1 to 3, **characterized in that** the NO synthase inhibiting substance and the phospholipase A2 inhibiting substance are used in separate form.

5. Use according to one of claims 1 to 3, **characterized in that** the NO synthase inhibiting substance and the phospholipase A2 inhibiting substance are in the form of a salt.

6. Use according to claim 5, **characterized in that** the salt is formed from a derivative of the NO synthase inhibiting substance containing at least one basic group and a derivative of the phospholipase A2 inhibiting substance containing at least one acid group.

7. Use according to one of the previous claims, **characterized in that**:
• the inhibitor(s) of NO synthases are chosen from the group constituted by:
- a compound of general formula I_{A} 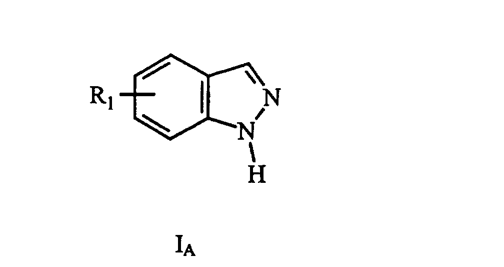 in which R₁ represents one or more substituents chosen from the hydrogen atom and the nitro, halo, alkyl or alkoxy radicals;
or a pharmaceutically acceptable salt of the latter;
- a compound of general formula II_{A} 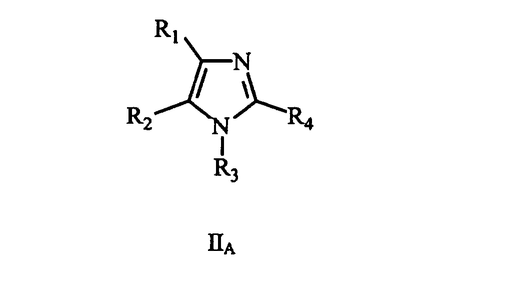 in which
R₁ and R₂ independently represent the hydrogen atom or the halo, hydroxy, amino, alkyl or alkoxy radical, or R₁ and R₂ are linked together and form a phenyl radical condensed with the imidazole ring, said phenyl radical being optionally substituted by one or more substituents chosen from the hydroxy, trifluoromethyl, halo, carboxy, alkyl, alkoxy or alkenyl radicals;
R₃ represents a hydrogen atom or an alkyl, amino, alkylamino or phenyl radical, the phenyl radical being optionally substituted by one or more substituents chosen from the hydroxy, trifluoromethyl, halo, carboxy, alkyl, lower alkoxy or alkenyl radicals;
and R₄ represents the hydrogen atom, an alkyl, amino or alkylamino radical;
or a pharmaceutically acceptable salt of the latter;
- a compound of general formula III_{A} 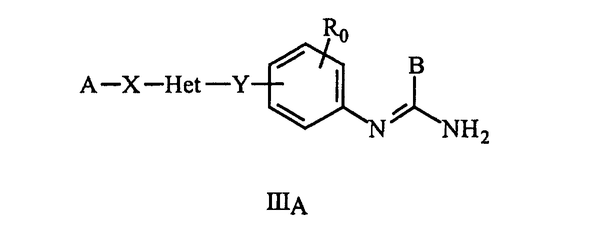
in which
R₀ represents H or an alkyl radical;
A is a 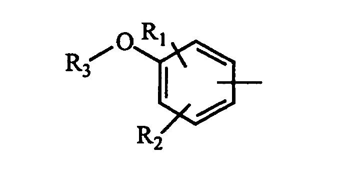 radical in which R₁ and R₂ represent, independently, a hydrogen atom, a halogen, the OH group, a linear or branched alkyl radical having 1 to 6 carbon atoms, a linear or branched alkoxy radical having 1 to 6 carbon atoms,
and R₃ represents a hydrogen atom, a linear or branched alkyl radical having 1 to 6 carbon atoms or a -COR₄ radical, R₄ representing an alkyl radical having 1 to 6 carbon atoms,
or a 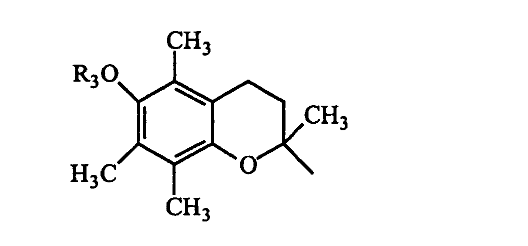 radical in which R₃ represents a hydrogen atom, a linear or branched alkyl radical having 1 to 6 carbon atoms or a -COR₄ radical, with R₄ representing an alkyl radical having 1 to 6 carbon atoms;
B represents a linear or branched alkyl radical having 1 to 6 carbon atoms, phenyl, pyridinyl or a heterocycle with 5 members containing 1 to 4 heteroatoms chosen from O, S, N and more particularly: thiophene, furane, pyrrole or thiazole, the carbons of which are optionally substituted by one or more groups chosen from a linear or branched alkyl radical having 1 to 6 carbon atoms, an alkoxy radical having 1 to 6 carbon atoms or a halogen;
X represents -CO-N(R₃)-X'-, -NH-CO-X'-, -CH=, -CO- or a bond, with X' representing -(CH₂)ₙ- where n is an integer from 0 to 6;
Y represents -Y'-, -CO-NH-Y',-Y'-NH-CO-, -CO-Y'-, -Y'-CO, -N(R₃)-Y'-, -Y'-N(R₃)-, Y'-CH₂-N(R₃)-CO-, -O-Y'-, -Y'-O-, -S-Y'-, -Y'-S-, -Y'-O-Y'-, -Y'-N(R₃)-Y'- or a bond, Y' representing -(CH₂)ₙ- where n is an integer from 0 to 6;
Het represents a heterocycle comprising 1 to 5 heteroatoms chosen from O, N, S being able to be substituted by one or more substituents X'-OR₃, X'-NR₃, X'-S-R₃ and such as for example :
oxetane, pyrrole, pyrrolidine, furane, tetrahydrofuran, thiophene, tetrahydrothiophene, sulpholane, piperazine, homopiperazine, 4-aminopiperidineimidazole, imidazoline, dihydroimidazole-2-one, dihydroimidazole-2-thione, oxazole, isoxazole, oxazoline, isoxazoline, oxazolidine, oxazolidinone, thiazole, thiazoline, thiazolidine, thiazolidinone, hydantoin, 1,2,4-triazole, 1,3,4-oxadiazole, 1,3,4-thiadiazole, 1,1-dioxide-1,2,5-thiadiazolidine, 1,2,4-triazole-3-one, tetrazole, tetrahydropyridine, azetidine;
or a pharmaceutically acceptable salt of the latter;
• the phospholipase A2 inhibitor(s) are chosen from the group constituted by mepacrine, aristolochic acid and their analogues, as well as the pharmaceutically acceptable salts of the latter.

8. Use according to one of claims 1 to 6, **characterized in that** the phospholipase A2 inhibitor(s) are chosen from the group constituted by:
• the compounds of general formula I_{B} or I'_{H} 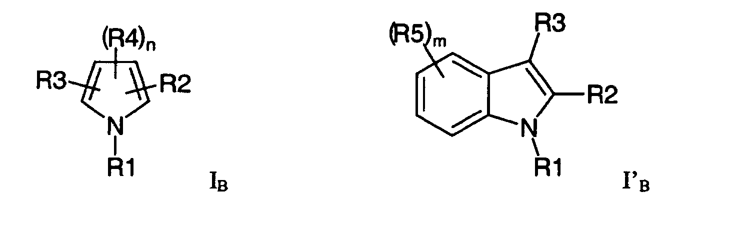 R1 represents a -Y1-Ar-Y2-Y3 group in which Y1 and Y2 independently represent a (C₁-C₁₂)-alkyl, (C₂-C₁₂)-alkenyl, (C₁-C₁₂)-alkoxy or (C₁-C₁₂)-alkenyloxy radical, the Y1 and Y2 radicals where appropriate being able to be interrupted by one or more oxygen atoms, Ar is an aryl group optionally substituted 1 to 3 times by substituents chosen independently from the R6, R7 and R8 groups, and Y3 represents a -COOR17, -CONR17R17, -CONHCOR19, -CONHS(O)₂R19, -CONHNHS(O)₂R19 or Tz radical;
R2 represents -COOR17, -Y4-COOR17, -CONR17R17, -Y4-CONR17R17, -CONHCOR19, -Y4-CONHCOR19, -CONHS(O)₂R19, -Y4-CONHS(O)₂R19, -CONHNHS(O)₂R19, -Y4-CONHNHS(O)₂R19, Tz or -Y4-Tz, with Y4 representing a (C₁-C₈)-alkyl, (C₂-C₈)-alkenyl radical, with the Y4 radical where appropriate being able to be interrupted by one or more oxygen atoms;
R3 represents a -CO-R9 radical in which R9 represents a -Y5, aryl or -Y5-aryl radical, with Y5 representing a (C₁-C₁₉)-alkyl, (C₂-C₁₉)-alkenyl or (C₂-C₁₉)-alkynyl radical, the Y5 radical where appropriate being able to be interrupted by one or more oxygen atoms and the aryl radical of R9 being optionally substituted 1 to 3 times by substituents chosen independently from the R10, R11 and R12 groups;
each R4 radical independently represents a hydrogen atom, a halogen atom, -CF₃, -Y6, aryl or -Y6-aryl, with Y6 representing a (C₁-C₈)-alkyl, (C₂-C₈)-alkenyl or (C₂-C₈)-alkynyl radical, the Y5 radical where appropriate being able to be interrupted by one or more oxygen atoms and the aryl radical of R4 being optionally substituted 1 to 3 times by substituents chosen independently from the R13, R14 and R15 groups and two neighbouring Y6 alkyl radicals being able to form together with the carbon atoms to which they are attached a ring with 5 to 8 members which can itself where appropriate be substituted by one or two (C₁-C₄)-alkyl radicals;
n represents the figure 2 and m represents the figure 4;
Tz is a 1H- or 2H-tetrazol-5-yl radical;
the R6, R7, R8, R10, R11, R12, R13, R14, R15 and R16 radicals are chosen independently from the group comprising the (C₁-C₂₀)-alkyl, (C₂-C₂₀)-alkenyl or (C₂-C₂₀)-alkynyl radicals optionally interrupted by one or more oxygen atoms, a halogen atom, the -CF₃, -CN, -NO₂ or -COCH₂OH groups and a perhalo(C₁-C₆)-alkenyl, -OR17, -SR17, -COOR17, -COR18, -NHCOR17, -NR17R17, -NHS(O)₂R17, -SOR17, -S(O)₂R17, -CONR17R17, -S(O)₂NR17R17, -OOCR18, -OOCNR17R17, -OOCOR17, -(CH₂)ᵣOR23, -(CH₂)ᵣSR23, -(CH₂)ᵣNHR23 or -(CH₂)ₛR20 radical;
R17 represents, independently each time it occurs, a -(CH₂)ₜR20 radical or one of the (C₁-C₂₀)-alkyl, (C₂-C₂₀)-alkenyl or (C₂-C₂₀)-alkynyl radicals optionally interrupted by one or more oxygen atoms;
R18 represents, independently each time it occurs, an R17, -CF₃, -(CH₂)ᵤCOOH or -(CH₂)ᵤCOOR21 radical;
R19 represents, independently each time it occurs, an R17 or -CF₃ radical;
R20 represents, independently each time it occurs, an aryl radical substituted by one or two R22 groups;
R21 represents, independently each time it occurs, a (C₁-C₆)-alkyl, benzyl or phenyl radical;
R22 represents, independently each time it occurs, a hydrogen atom, a halogen atom, a (C₁-C₁₂)-alkyl, (C₁-C₁₂)-alkoxy, (C₁-C₁₂)-alkylthio, (C₁-C₁₂)-alkylsulphonyl, (C₁-C₁₂)-alkylcarbonyl, -CF₃, -CN or -NO₂ radical;
R23 represents, independently each time it occurs, a hydrogen atom or a -COR21 radical;
r is an integer from 1 to 20;
s and t represent independently integers from 0 to 12;
and u represents an integer from 0 to 4;
• the compounds of general formula II_{B} 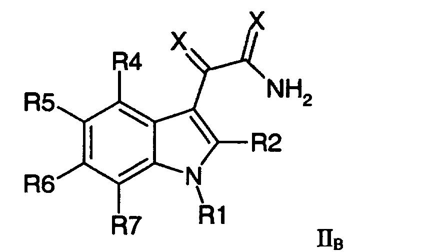 in which
the two X groups represent oxygen atoms;
R1 is chosen from the group constituted by the 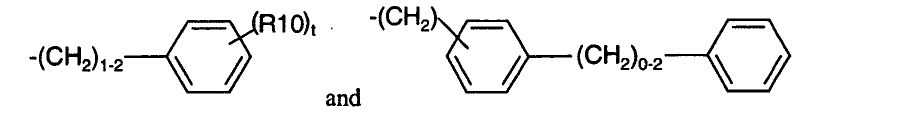 radicals, in which R10 is chosen independently from a halogen atom and a (C₁-C₁₀)-alkyl, (C₁-C₁₀)-alkoxy, (C₁-C₁₀)-alkylthio and (C₁-C₁₀)-haloalkyl radical and t is an integer from 0 to 5;
R2 is chosen from a halogen atom and a cyclopropyl, methyl, ethyl or propyl radical;
R4 and R5 are chosen independently from a hydrogen atom, a substituent which does not interfere or the (acid) -Lₐ-(acid group) group, it being understood that -Lₐ- for R4 is chosen from -O-CH₂-, -S-CH₂-, -NH-CH₂-, -CH₂-CH₂-, -O-CH(CH₃)- and -O-CH(CH₂-CH₂-Ph)-, Ph being a phenyl radical and that -Lₐ- for R5 is chosen from the 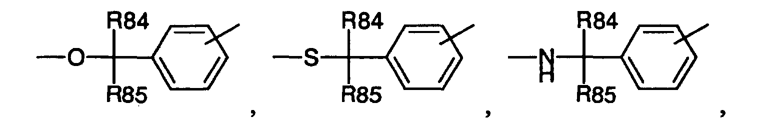 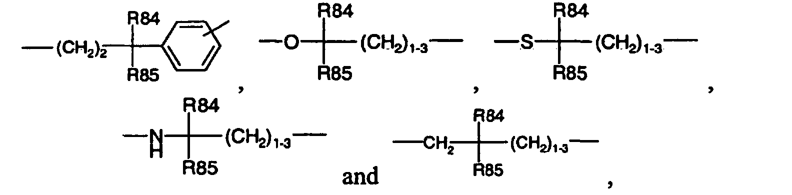 radicals in which R84 and R85 are chosen independently from a hydrogen atom, a halogen atom and a (C₁-C₁₀)-alkyl, aryl, (C₁-C₁₀)-alkaryl, (C₁-C₁₀)-aralkyl, carboxy or carbalkoxy radical,
it being understood also that at least one of R4 and R5 must be the -Lₐ-(acid group) group and that the (acid group) of R4 and R5 is chosen from -CO₂H, -SO₃H and -P(O)(OH)₂;
R6 and R7 are chosen independently from a hydrogen atom and a substituent which does not interfere;
substituents which do not interfere being chosen independently from the group comprising the (C₁-C₆)-alkyl, (C₂-C₆)-alkenyl, (C₂-C₆)-alkynyl, (C₇-C₁₈)-aralkyl, (C₇-C₁₈)-alkaryl, (C₃-C₈)-cycloalkyl, (C₃-C₈)-cycloalkenyl, phenyl, toluyl, xylenyl, phenylmethyl, (C₁-C₆)-alkoxy, (C₂-C₆)-alkenyloxy, (C₂-C₆)-alkynyloxy, (C₂-C₁₂)-alkoxyalkyl, (C₂-C₁₂)-alkoxyalkyloxy, (C₂-C₁₂)-alkylcarbonylamino, (C₂-C₁₂)-alkoxyamino, (C₂-C₁₂)-alkoxyaminocarbonyl, (C₂-C₁₂)-alkylamino, (C₁-C₆)-alkylthio, (C₂-C₁₂)-alkylthiocarbonyl, (C₁-C₆)-alkylsulphinyl, (C₁-C₆)-alkylsulphonyl, (C₂-C₆)-haloalkoxy, (C₂-C₆)-haloalkylsulphonyl, (C₂-C₆)-haloalkyl, (C₁-C₆)-hydroxyalkyl, - C(O)O((C₁-C₆)-alkyl), -(CH₂)ₙ-O-((C₁-C₆)-alkyl), benzyloxy, phenoxy, phenylthio, - CHO, amino, amidino, bromo, carbamyl, carboxyl, carbalkoxy, -(CH₂)ₙ-CO₂H, chloro, cyano, cyanoguanidinyl, fluoro, guanidino, hydrazide, hydrazino, hydrazido, hydroxy, hydroxyamino, iodo, nitro, phosphono, -SO₃H, thioacetal, thiocarbonyl and (C₁-C₆)-alkylcarbonyl radicals, n being an integer from 1 to 8;
• the compounds of general formula III_{B} 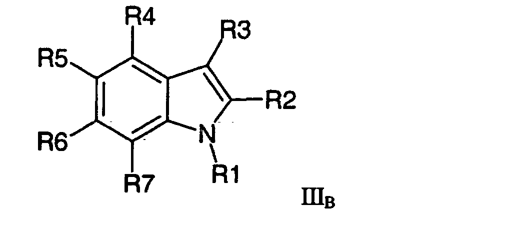 in which
R1 is chosen from groups (a), (b) and (c);
(a) represents a (C₇-C₂₀)-alkyl, (C₇-C₂₀)-haloalkyl, (C₇-C₂₀)-alkenyl or (C₇-C₂₀)-alkynyl radical, a saturated or unsaturated carbon ring with 5 to 14 members optionally substituted by one or more substituents chosen from substituents which do not interfere, or also (a) represents a heterocycle with 5 to 14 members containing 1 to 3 heteroatoms, said heterocycle being saturated or unsaturated and optionally substituted by one or more substituents chosen from substituents which do not interfere;
(b) is one of the (a) radicals substituted by one or more substituents chosen from substituents which do not interfere;
(c) represents the -(L₁)-R11 group in which -(L₁)- is a group chosen from the groups represented by the formulae 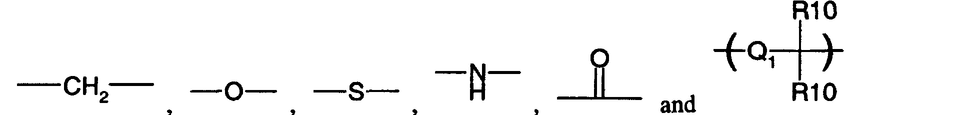 in which Q₁ represents a bond or one of the -CH₂-, -O-, -S-, -NH- and -CO- groups and each of the R10 groups independently represents a hydrogen atom or a (C₁-C₈)-alkyl, (C₁-C₈)-haloalkyl or (C₁-C₈)-alkoxy radical,
and R11 is a group chosen from groups (a) and (b);
R2 is a hydrogen atom, a halogen atom, or a (C₁-C₄)-alkyl, (C₂-C₄)-alkenyl, -O-(C₁-C₃)-alkyl, -S-(C₁-C₃)-alkyl, -(C₁-C₄)-cycloalkyl, -CF₃, -NO₂, -CN or -SO₃ radical;
R3 represents -(L₃)-Z in which -(L₃)- is a divalent bond group chosen from a bond and the -CH₂-, -O-, -S-, -NH- and -CO- groups;
and Z is chosen from the acetamide, thioacetamide, glyoxylamide, thioglioxylamide, hydrazide or thiohydrazide groups represented by the general formulae 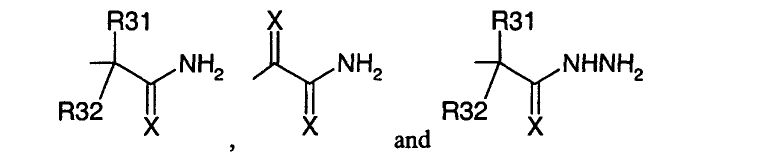 in which R31 and R32 are chosen independently from a hydrogen atom and a (C₁-C₈)-alkyl, (C₁-C₈)-haloalkyl or (C₃-C₄)-cycloalkyl radical and X represents, independently each time it occurs, an oxygen or sulphur atom;
R4 and R5 are chosen independently from a hydrogen atom, a substituent which does not interfere or the -(Lₐ)-(acylsulphonamide group) group in which -(Lₐ)- for R4 is a group represented by the formula 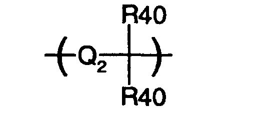 in which Q₂ is chosen from -CH₂-, -O-, -NH-, -C(O)- and -S-, and each of the R40 groups is chosen independently from a hydrogen atom, a halogen atom and the (C₁-C₈)-alkyl, aryl, (C₁-C₈)-alkaryl, (C₁-C₈)-alkoxy and aralkyl radicals,
-(Lₐ)- for R5 is chosen from the groups represented by the formulae 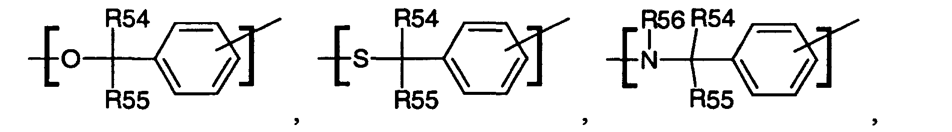 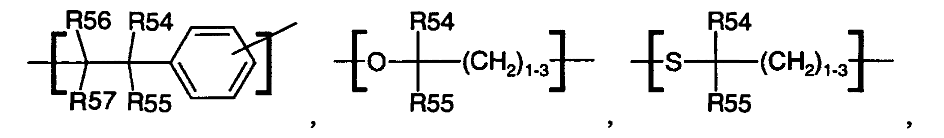 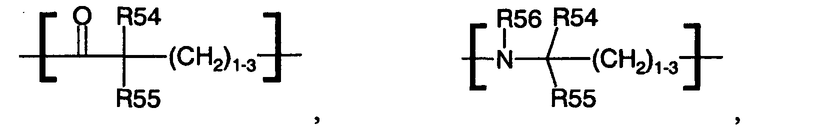 and 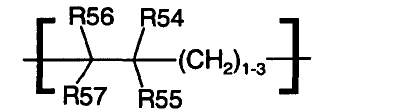 in which R54, R55, R56 and R57 independently are chosen from the group comprising a hydrogen atom, a halogen atom and the (C₁-C₈)-alkyl, (C₁-C₈)-alkoxy, (C₁-C₈)-haloalkyl and aryl radicals,
the (acylsulphonamide) group being a group of formula -CO-NH-SO₂-R81 in which R81 is chosen from the group comprising the -CF₃, (C₁-C₈)-alkyl, (C₁-C₈)-alkoxy, (C₁-C₈)-alkylthio, (C₁-C₈)-alkylamino, (C₁-C₈)-haloalkyl, (C₁-C₁₄)-aralkyl, (C₁-C₁₄)-alkylaryl, aryl, thioaryl, (C₃-C₁₄)-cycloalkyl radicals and a heterocycle with 3 to 14 members, said heterocycle containing 1 to 3 heteroatoms and being saturated or unsaturated,
it being understood moreover that at least one of R4 and R5 is an -(Lₐ)-(acylsulphonamide group) group;
R6 and R7 independently represent a hydrogen atom, a substituent which does not interfere, a saturated or unsaturated carbon ring with 5 to 14 members optionally substituted by one or more substituents chosen from substituents which do not interfere or a heterocycle with 5 to 14 members containing 1 to 3 heteroatoms, said heterocycle being saturated or unsaturated and optionally substituted by one or more substituents chosen from substituents which do not interfere;
substituents which do not interfere being chosen independently from the group comprising the (C₁-C₈)-alkyl, (C₂-C₈)-alkenyl, (C₂-C₈)-alkynyl, (C₇-C₁₂)-aralkyl, (C₇-C₁₂)-alkaryl, (C₃-C₈)-cycloalkyl, (C₃-C₈)-cycloalkenyl, phenyl, toluyl, xylenyl, biphenyl, (C₁-C₈)-alkoxy, (C₂-C₈)-alkenyloxy, (C₂-C₈)-alkynyloxy, (C₂-C₁₂)-alkoxyalkyl, (C₂-C₁₂)-alkoxyalkyloxy, (C₂-C₁₂)-alkylcarbonyl, (C₂-C₁₂)-alkylcarbonylamino, (C₂-C₁₂)-alkoxyamino, (C₂-C₁₂)-alkoxyaminocarbonyl, (C₁-C₁₂)-alkylamino, (C₁-C₆)-alkylthio, (C₂-C₁₂)-alkylthiocarbonyl, (C₁-C₈)-alkylsulphinyl, (C₁-C₈)-alkylsulphonyl, (C₂-C₈)-haloalkoxy, (C₁-C₈)-haloalkylsulphonyl, (C₂-C₈)-haloalkyl, (C₁-C₈)-hydroxyalkyl, -C(O)O((C₁-C₈)-alkyl), -(CH₂)ₙ-O-((C₁-C₈)-alkyl), benzyloxy, phenoxy, phenylthio, -CONHSO₂R, -CHO, amino, amidino, bromo, carbamyl, carboxyl, carbalkoxy, -(CH₂)ₙ-CO₂H, chloro, cyano, cyanoguanidinyl, fluoro, guanidino, hydrazide, hydrazino, hydrazido, hydroxy, hydroxyamino, iodo, nitro, phosphono, -SO₃H, thioacetal, thiocarbonyl radicals, n being an integer from 1 to 8 and R being a (C₁-C₈)-alkyl radical;
• the compounds of general formula IV_{B} 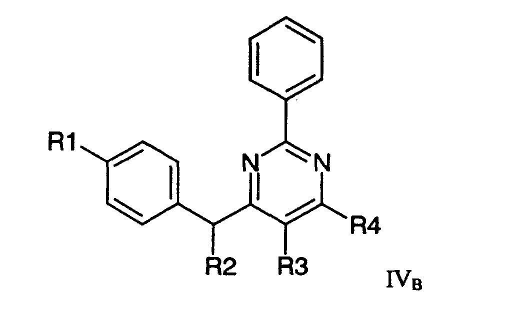 in which
R1 is chosen from the group comprising a halogen atom and the (C₁-C₆)-alkyl or (C₁-C₆)-alkoxy radicals;
R2 is chosen from the group comprising one of the phenyl or benzyl radicals optionally substituted on the aromatic ring by a halogen atom or a (C₁-C₆)-alkyl, (C₁-C₆)-alkoxy, amino, alkylamino, dialkylamino, carboxyl, carbamoyl, (C₁-C₈)-acyl, sulphonyl, thiol or alkylthio radical, and a (C₃-C₇)-cycloalkyl radical, said (C₃-C₇)-cycloalkyl radical being optionally condensed with a benzene ring or a heterocycle and being optionally substituted by a halogen atom or a (C₁-C₆)-alkyl radical;
R3 is chosen from the group comprising a halogen atom and a (C₁-C₆)-alkyl radical;
and R4 is chosen from the group comprising -H, -OH, -N₃ and -NHCOCH_{3;}
• and the pharmaceutically acceptable salts of the compounds of general formula I_{B}, II_{B}, III_{B} or IV_{B}.

9. Use according to claim 1, **characterized in that**:
• the NO synthase inhibitor(s) are chosen from the group constituted by L-nitro-arginine (LNA), the L-nitro-arginine methyl ester (LNAME), L-N-monomethylarginine (LNMMA), aminoguanidine, agmatine, 2-amino-1-(methylamino)benzimidazole, 5-nitro-indazole, 6-nitro-indazole, 7-nitro-indazole, 1,2-(trifluoromethylphenyl)imidazole (TRIM), 2-amino-4-methyl-6-(2-aminoethyl)pyridine, 2-iminopiperidine, 2-iminohomopiperidine, 2-imino-5,6-dihydro-1,3-thiazine, 2-imino-5,6-dihydro-1,3-oxazine, N-phenyl-2-thiophenecarboximidamide, 2-iminotetrahydropyrimidine, S-ethylisothiourea, S-methyl-L-thiocitrulline, S-ethyl-L-thiocitrulline, (S)-N- {4-[4-[(3,4-dihydro-6-hydroxy-2,5,7,8-tetramethyl-2H-1-benzopyran-2-yl)-carbonyl]-1-piperazinyl]phenyl}-2-thiophenecarboximidamide and 4-[3,5-bis(1,1-dimethylethyl)-4-hydroxyphenyl]-N-{4-[(imino(2-thienyl)methyl)amino]phenyl}-N-methyl-2-thiazolemethanamine, as well as the pharmaceutically acceptable salts of the latter; and
• the phospholipases A2 inhibitor(s) are chosen from the group constituted by mepacrine and aristolochic acid and their analogues, as well as the pharmaceutically acceptable salts of the latter.

10. Use according to claim 9, **characterized in that**:
• the NO synthase inhibitor(s) are chosen from the group constituted by aminoguanidine, 7-nitro-indazole and 4-[3,5-bis(1,1-dimethylethyl)-4-hydroxyphenyl]-N- {4-[(imino(2-thienyl)methyl)amino]phenyl}-N-methyl-2-thiazole-methanamine, as well as the pharmaceutically acceptable salts of the aforementioned compounds; and
• the phospholipase A2 inhibitor(s) are chosen from the group constituted by mepacrine and aristolochic acid and their analogues, as well as the pharmaceutically acceptable salts of the latter.

11. Use according to one of claims 1 to 10, **characterized in that** the pathologies or disorders are chosen from proliferative and inflammatory diseases such as atherosclerosis, pulmonary hypertension, glomerulonephritis, portal hypertension, psoriasis, arthrosis and rheumatoid arthritis, fibroses, amyloidoses and inflammations of the gastrointestinal system or the pulmonary system and airways.

12. Use according to one of claims 1 to 10, **characterized in that** the pathologies or disorders are chosen from autoimmune and viral diseases such as lupus, AIDS, parasitic and viral infections, diabetes, multiple sclerosis or myopathies.

## Patentansprüche

1. Verwendung eines Produkts, das mindestens eine NO-Synthasen hemmende Substanz in Verbindung mit mindestens einer Phospholipasen A2 hemmenden Substanz in getrennter Form oder in Kombination enthält, zur Herstellung eines Medikaments, das zur Behandlung einer Krankheit oder einer Störung bestimmt ist, die aus kardiovaskulären und zerebrovaskulären Störungen, Störungen des Zentralnervensystems oder des peripheren Nervensystems, proliferativen und entzündlichen Krankheiten, Diarrhoe, Erbrechen, radioaktiven Bestrahlungen, Sonnenbestrahlung, Organtransplantationen, Autoimmun- und Viruserkrankungen und Krebs ausgewählt sind.

2. Verwendung nach Anspruch 1, **dadurch gekennzeichnet, daß** die NO-Synthasen hemmende Substanz eine die neuronale Form der NO-Synthasen hemmende Substanz ist.

3. Verwendung nach Anspruch 1, **dadurch gekennzeichnet, daß** die NO-Synthasen hemmende Substanz eine die induzierbare Form der NO-Synthasen hemmende Substanz ist.

4. Verwendung nach einem der Ansprüche 1 bis 3, **dadurch gekennzeichnet, daß** die NO-Synthasen hemmende Substanz und die Phospholipasen A2 hemmende Substanz in getrennter Form verwendet werden.

5. Verwendung nach einem der Ansprüche 1 bis 3, **dadurch gekennzeichnet, daß** die NO-Synthasen hemmende Substanz und die Phospholipasen A2 hemmende Substanz in Form von Salz vorliegen.

6. Verwendung nach Anspruch 5, **dadurch gekennzeichnet, daß** das Salz ausgehend von einem Derivat der NO-Synthasen hemmenden Substanz, das mindestens eine basische Gruppe enthält, und von einem Derivat der Phospholipasen A2 hemmenden Substanz, das mindestens eine saure Gruppe enthält, gebildet ist.

7. Verwendung nach einem der vorhergehenden Ansprüche, **dadurch gekennzeichnet, daß**
• der oder die NO-Synthasen-Hemmer aus der Gruppe ausgewählt sind, die besteht aus:
- einer Verbindung der allgemeinen Formel I_{A} 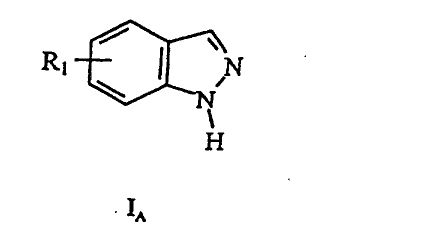 in der R₁ einen oder mehrere Substituenten darstellt, die aus einem Wasserstoffatom und den Resten Nitro, Halogen, Alkyl oder Alkoxy ausgewählt sind;
oder einem pharmazeutisch verträglichen Salz davon;
- einer Verbindung der allgemeinen Formel II_{A} 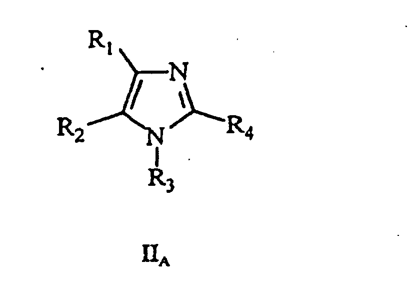 in der
R₁ und R₂ unabhängig voneinander ein Wasserstoffatom oder einen der Reste Halogen, Hydroxy, Amino, Alkyl oder Alkoxy darstellen oder R₁ und R₂ miteinander verbunden sind und einen an den Imidazolring kondensierten Phenylrest bilden,
wobei dieser Phenylrest ggf. durch einen oder mehrere Substituenten substituiert ist, die aus den Resten Hydroxy, Trifluormethyl, Halogen, Carboxy, Alkyl, Alkoxy oder Alkenyl ausgewählt sind;
R₃ ein Wasserstoffatom oder einen der Reste Alkyl, Amino, Alkylamino oder Phenyl darstellt, wobei der Phenylrest ggf. durch einen oder mehrere Substituenten substituiert ist, die aus den Resten Hydroxy, Trifluormethyl, Halogen, Carboxy, Alkyl, Niederalkoxy oder Alkenyl ausgewählt sind; und R4 ein Wasserstoffatom, einen Alkyl-, Amino- oder Alkylaminorest darstellt;
oder einem pharmazeutisch verträglichen Salz davon;
- einer Verbindung der allgemeinen Formel III_{A} 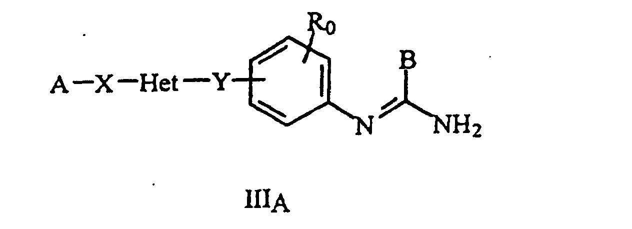 in der
R₀ H oder einen Alkylrest darstellt;
A ein Rest 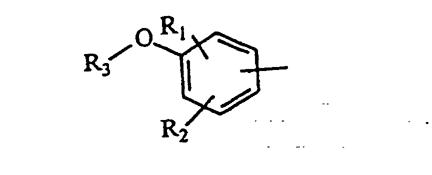 ist, in dem R₁ und R₂ unabhängig voneinander ein Wasserstoffatom, ein Halogen, eine OH-Gruppe, einen linearen oder verzweigten Alkylrest mit 1 bis 6 Kohlenstoffatomen, einen linearen oder verzweigten Alkoxyrest mit 1 bis 6 Kohlenstoffatomen darstellen,
und R₃ ein Wasserstoffatom, einen linearen oder verzweigten Alkylrest mit 1 bis 6 Kohlenstoffatomen oder einen Rest -COR₄ darstellt, wobei R₄ einen Alkylrest mit 1 bis 6 Kohlenstoffatomen darstellt,
oder ein Rest 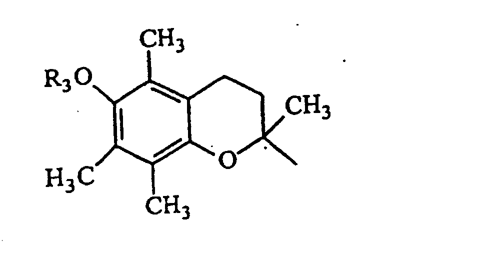 in dem R₃ ein Wasserstoffatom, einen linearen oder verzweigten Alkylrest mit 1 bis 6 Kohlenstoffatomen oder einen Rest -COR₄ darstellt, wobei R₄ einen Alkylrest mit 1 bis 6 Kohlenstoffatomen darstellt;
B folgendes darstellt: einen linearen oder verzweigten Alkylrest mit 1 bis 6 Kohlenstoffatomen, Phenyl, Pyridinyl oder einen Heterocyclus mit 5 Kettengliedern, der 1 bis 4 Heteroatome enthält, die aus O, S, N ausgewählt sind, und insbesondere: Thiophen, Furan, Pyrrol oder Thiazol, deren Kohlenstoffatome ggf. durch eine oder mehrere Gruppen substituiert sind, die aus einem linearen oder verzweigten Alkylrest mit 1 bis 6 Kohlenstoffatomen, einem Alkoxyrest mit 1 bis 6 Kohlenstoffatomen oder Halogen ausgewählt sind;
X -CO-N(R₃)-X'-, -NH-CO-X'-, -CH=, -CO- oder eine Bindung darstellt, wobei X' -(CH₂)ₙ- darstellt, wobei n eine ganze Zahl von 0 bis 6 ist;
Y -Y'-, -CO-NH-Y', -Y'-NH-CO-, -CO-Y'-, -Y'-CO, -N(R₃)-Y'-, -Y'-N(R₃)-, Y'-CH₂-N-(R₃)-CO-, -O-Y'-, -Y'-O-, -S-Y'-, -Y'-S-, -Y'-O-Y'-, -Y'-N(R₃)-Y'- oder eine Bindung darstellt, wobei Y' -(CH₂)ₙ- darstellt, wobei n eine ganze Zahl von 0 bis 6 ist;
Het einen Heterocyclus mit 1 bis 5 Heteroatomen darstellt, die aus O, N, S ausgewählt sind, die durch einen oder mehrere Substituenten X'-OR₃, X'-NR₃, X'-S-R₃ substituiert sein können, wie z.B.:
Oxetan, Pyrrol, Pyrrolidin, Furan, Tetrahydrofuran, Thiophen, Tetrahydrothiophen, Sulfolan, Piperazin, Homopiperazin, 4-Aminopiperidinimidazol, Imidazolin, Dihydroimidazol-2-on, Dihydroimidazol-2-thion, Oxazol, Isoxazol, Oxazolin, Isoxazolin, Oxazolidin, Oxazolidinon, Thiazol, Thiazolin, Thiazolidin, Thiazolidinon, Hydantoin, 1,2,4-Triazol, 1,3,4-Oxadiazol, 1,3,4-Thiadiazol, 1,1-Dioxid-1,2,5-thiadiazolidin, 1,2,4-Triazol-3-on, Tetrazol, Tetrahydropyridin, Azetidin;
oder einem pharmazeutisch verträglichen Salz davon;
• der oder die Phospholipasen-A2-Hemmer aus der Gruppe ausgewählt sind, die aus Mepacrin, Aristolochiasäure und ihren Analogen sowie den pharmazeutisch verträglichen Salzen davon ausgewählt sind.

8. Verwendung nach einem der Ansprüche 1 bis 6, **dadurch gekennzeichnet, daß** der oder die Phospholipasen-A2-Hemmer aus der Gruppe ausgewählt sind, die besteht aus:
• den Verbindungen der allgemeinen Formel I_{B} oder I'_{B} 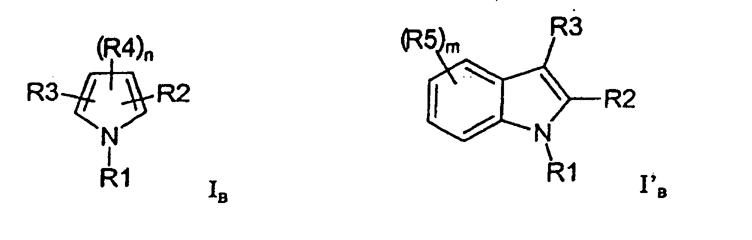 in denen
R1 eine Gruppe -Y1-Ar-Y2-Y3 darstellt, in der Y1 und Y2 unabhängig voneinander einen Rest (C₁-C₁₂)-Alkyl, (C₂-C₁₂)-Alkenyl, (C₁-C₁₂)-Alkoxy oder (C₁-C₁₂)-Alkenyloxy darstellen, wobei die Reste Y1 und Y2 ggf. durch ein oder mehrere Sauerstoffatome unterbrochen sein können, Ar eine Arylgruppe ist, die ggf. 1 bis 3mal durch Substituenten substituiert ist, die unabhängig voneinander aus den Gruppen R6, R7 und R8 ausgewählt sind, und Y3 einen Rest -COOR17, -CONR17R17, -CONHCOR19, - CONHS(O)₂R19, -CONHNHS(O)₂R19 oder Tz darstellt;
R2 -COOR17, -Y4-COOR17, -CONR17R17, -Y4-CONR17R17, -CONHCOR19, -Y4-CONHCOR19, -CONH(O)₂R19, -Y4-CONHS(O)₂R19, -CONHNHS(O)₂R19, -Y4-CONHNHS(O)₂R19, Tz oder -Y4-Tz darstellt, wobei Y4 einen Rest (C₁-C₈)-Alkyl, (C₂-C₈)-Alkenyl darstellt, wobei der Rest Y4 ggf. durch ein oder mehrere Sauerstoffatome unterbrochen sein kann;
R3 einen Rest -CO-R9 darstellt, in dem R9 einen Rest -Y5, Aryl oder -Y5-Aryl darstellt, wobei Y5 einen Rest (C₁-C₁₉) -Alkyl, (C₂-C₁₉)-Alkenyl oder (C₂-C₁₉)-Alkinyl darstellt, wobei der Rest Y5 ggf. durch ein oder mehrere Sauerstoffatome unterbrochen sein kann und der Arylrest von R9 ggf. 1 bis 3mal durch Substituenten substituiert ist, die unabhängig voneinander aus den Gruppen R10, R11 und R12 ausgewählt sind;
jeder Rest R4 unabhängig voneinander ein Wasserstoffatom, ein Halogenatom, -CF₃, -Y6, Aryl oder -Y6-Aryl darstellt, wobei Y6 einen Rest (C₁-C₈)-Alkyl, (C₂-C₈)-Alkeny oder (C₂-C₈)-Alkinyl darstellt, wobei der Rest Y5 ggf. durch ein oder mehrere Sauerstoffatome unterbrochen sein kann und der Arylrest von R4 ggf. 1 bis 3mal durch Substituenten substituiert ist, die unabhängig voneinander aus den Gruppen R13, R14 und R15 ausgewählt sind, und zwei benachbarte Alkylreste Y6 zusammen mit den Kohlenstoffatomen, an die sie gebunden sind, einen Ring mit 5 bis 8 Kettengliedern bilden können, der seinerseits ggf. durch einen oder zwei Reste (C₁-C₄)-Alkyl substituiert sein kann;
n die Zahl 2 und m die Zahl 4 darstellt;
Tz ein Rest 1H- oder 2H-Tetrazol-5-yl ist;
die Reste R6, R7, R8, R10, R11, R12, R13, R14, R15 und R16 unabhängig voneinander aus der Gruppe ausgewählt sind, die aus den folgenden Resten bestehen: (C₁-C₂₀)-Alkyl, (C₂-C₂₀)-Alkenyl oder (C₂-C₂₀)-Alkinyl, die ggf. durch ein oder mehrere Sauerstoffatome unterbrochen sind, einem Halogenatom, den Gruppen -CF₃, -CN, NO₂ oder -COCH₂OH und einem der Reste Perhalogen(C₁-C₆)-alkenyl, -OR17, -SR17, -COOR17, -COR18, -NHCOR17, -NR17R17, -NHS(O)₂R17, -SOR17, -S(O)₂R17, -CONR17R17, -S(O)₂NR17R17, -OOCR18, -OOCNR17R17, -OOCOR17, -(CH₂)ᵣOR23, -(CH₂)ᵣSR23, -(CH₂)ᵣNHR23 oder -(CH₂)ₛR20; R17 jedesmal, wenn es auftritt, unabhängig voneinander einen Rest -(CH₂)ₜR20 oder einen der Reste (C₁-C₂₀)-Alkyl, (C₂-C₂₀)-Alkenyl oder (C₂-C₂₀)-Alkinyl darstellt, die ggf. durch ein oder mehrere Sauerstoffatome unterbrochen sind; R18 jedesmal, wenn es auftritt, unabhängig voneinander einen Rest R17, -CF₃, -(CH_{z})ᵤCOOH oder -(CH₂)ᵤCOOR21 darstellt;
R19 jedesmal, wenn es auftritt, unabhängig voneinander einen Rest R17 oder -CF₃ darstellt;
R20 jedesmal, wenn es auftritt, unabhängig voneinander einen durch eine oder zwei Gruppen R22 substituierten Arylrest darstellt;
R21 jedesmal, wenn es auftritt, unabhängig voneinander einen Rest (C₁-C₆)-Alkyl, Benzyl oder Phenyl darstellt;
R22 jedesmal, wenn es auftritt, unabhängig voneinander ein Wasserstoffatom, ein Halogenatom, einen Rest (C₁-C₁₂)-Alkyl, (C₁-C₁₂)-Alkoxy, (C₁-C₁₂)-Alkylthio, (C₁-C₁₂)-Alkylsulfonyl, (C₁-C₁₂)-Alkylcarbonyl, -CF₃, -CN oder -NO₂ darstellt;
R23 jedesmal, wenn es auftritt, unabhängig voneinander ein Wasserstoffatom oder einen Rest -COR21 darstellt;
r eine ganze Zahl von 1 bis 20 ist;
s und t unabhängig voneinander ganze Zahlen von 0 bis 12 darstellen;
und u eine ganze Zahl von 0 bis 4 darstellt;
• den Verbindungen der allgemeinen Formel II_{B} 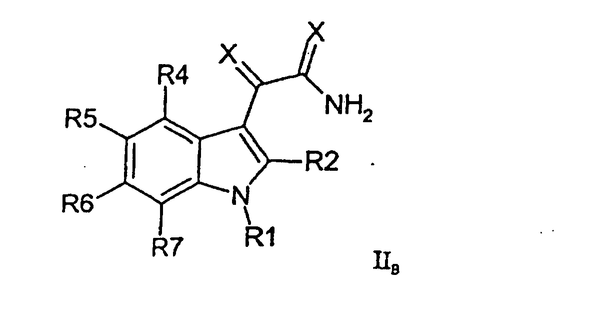 in der
die beiden Gruppen X Sauerstoffatome darstellen; R1 aus der Gruppe ausgewählt ist, die aus den Resten 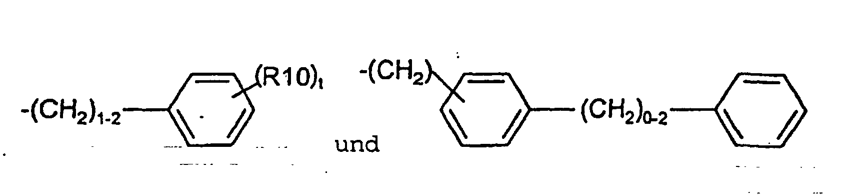 besteht, in denen R10 unabhängig voneinander aus einem Halogenatom und einem Rest (C₁-C₁₀)-Alkyl, (C₁-C₁₀)-Alkoxy, (C₁-C₁₀)-Alkylthio, (C₁-C₁₀)-Halogenalkyl ausgewählt ist und
t eine ganze Zahl von 0 bis 5 ist;
R2 aus einem Halogenatom und einem Rest Cyclopropyl, Methyl, Ethyl oder Propyl ausgewählt ist;
R4 und R5 unabhängig voneinander aus einem Wasserstoffatom, einem nicht störenden Substituenten oder der Gruppe (Säure)-Lₐ-(saure Gruppe) ausgewählt sind, wobei gilt, daß -Lₐbei R4 aus -O-CH₂, -S-CH₂-, -NH-CH₂-, -CH₂-CH₂-, -O-CH(CH₃)-und -O-CH(CH₂-CH₂-Ph)- ausgewählt ist, wobei Ph ein Phenylrest ist, und -Lₐ- bei R5 aus den Resten 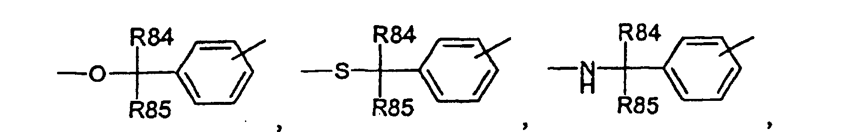 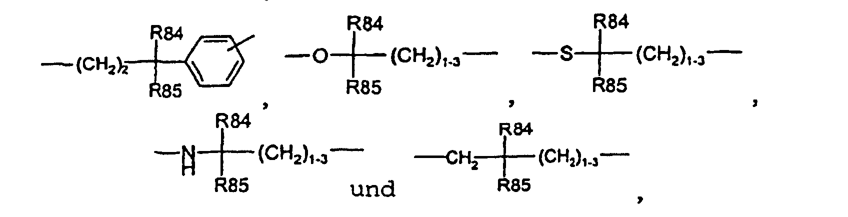 ausgewählt ist, in denen R84 und R85 unabhängig voneinander aus einem Wasserstoffatom, einem Halogenatom und einem Rest (C₁-C₁₀)-Alkyl, Aryl, (C₁-C₁₀)-Alkaryl, (C₁-C₁₀)-Aralkyl, Carboxy oder Carbalkoxy ausgewählt sind,
wobei ferner gilt, daß mindestens einer von R4 und R5 die Gruppe -Lₐ-(saure Gruppe) sein muß und daß die (saure Gruppe) von R4 und R5 aus -CO₂H, -SO₃H und -P(O)(OH)₂ ausgewählt ist;
R6 und R7 unabhängig voneinander aus einem Wasserstoffatom und einem nicht störenden Substituenten ausgewählt sind; wobei die nicht störenden Substituenten voneinander unabhängig aus der Gruppe ausgewählt sind, die aus den folgenden Resten besteht: (C₁-C₆)-Alkyl, (C₂-C₆)-Alkenyl, (C₂-C₆)-Alkinyl, (C₇-C₁₈)-Aralkyl, (C₇-C₁₈)-Alkaryl, (C₃-C₈)-Cycloalkyl, (C₃-C₈)-Cycloalkenyl, Phenyl, Toluyl, Xylenyl, Phenylmethyl, (C₁-C₆)-Alkoxy, (C₂-C₆)-Alkenyloxy, (C₂-C₆)-Alkinyloxy, (C₂-C₁₂)-Alkoxyalkyl, (C₂-C₁₂)-Alkoxyalkyloxy, (C₂-C₁₂)-Alkylcarbonylamino, (C₂-C₁₂)-Alkoxyamino, (C₂-C₁₂)-Alkoxyaminocarbonyl, (C₂-C₁₂)-Alkylamino, (C₁-C₆)-Alkylthio, (C₂-C₁₂)-Alkylthiocarbonyl, (C₁-C₆)-Alkylsulfinyl, (C₁-C₆)-Alkylsulfonyl, (C₂-C₆)-Halogenalkoxy, (C₂-C₆)-Halogenalkylsulfonyl, (C₂-C₆)-Halogenalkyl, (C₁-C₆)-Hydroxyalkyl, -C(O)O((C₁-C₆)-Alkyl), -(CH₂)ₙ-O-((C₁-C₆)-Alkyl), Benzyloxy, Phenoxy, Phenylthio, -CHO, Amino, Amidino, Brom, Carbamyl, Carboxyl, Carbalkoxy, -(CH₂)ₙ-CO₂H, Chlor, Cyan, Cyanguanidinyl, Fluor, Guanidino, Hydrazid, Hydrazino, Hydrazido, Hydroxy, Hydroxyamino, Iod, Nitro, Phosphono, -SO₃H, Thioacetal, Thiocarbonyl und (C₁-C₆)-Alkylcarbonyl, wobei n eine ganze Zahl von 1 bis 8 ist;
• den Verbindungen der allgemeinen Formel III_{B} 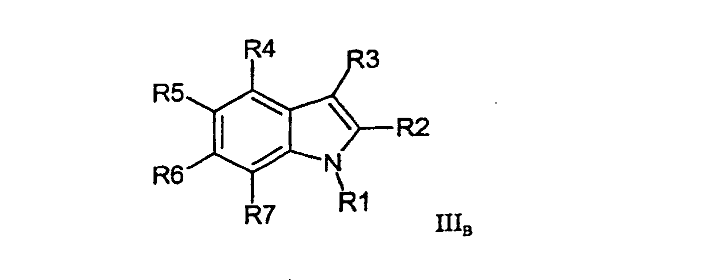 in der
R1 aus den Gruppen (a), (b) und (c) ausgewählt ist; wobei (a) einen Rest (C₇-C₂₀)-Alkyl, (C₇-C₂₀)-Halogenalkyl, (C₇-C₂₀)-Alkenyl oder (C₇-C₂₀)-Alkinyl, einen gesättigten oder ungesättigten kohlenstoffhaltigen Ring mit 5 bis 14 Kettengliedern, der ggf. durch einen oder mehrere Substituenten substituiert ist, die aus den nicht störenden Substituenten ausgewählt sind, oder (a) einen Heterocyclus mit 5 bis 14 Kettengliedern darstellt, der 1 bis 3 Heteroatome enthält, wobei dieser Heterocyclus gesättigt oder ungesättigt ist und ggf. durch einen oder mehrere Substituenten substituiert ist, die aus den nicht störenden Substituenten ausgewählt sind;
(b) einer der Reste (a) ist, der durch einen oder mehrere Substituenten substituiert ist, die aus den nicht störenden Substituenten ausgewählt sind;
(c) die Gruppe -(L₁)-R11 darstellt, in der -(L₁)- eine Gruppe ist, die aus den Gruppen ausgewählt ist, die durch die Formeln 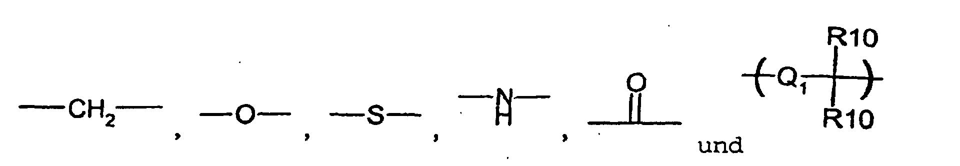 dargestellt sind, in denen Q₁ eine Bindung oder eine der Gruppen -CH₂-, -O-, -S-, -NH- und -CO- darstellt und jede der Gruppen R10 unabhängig voneinander ein Wasserstoffatom oder einen der Reste (C₁-C₈)-Alkyl, (C₁-C₈)-Halogenalkyl, (C₁-C₈)-Alkoxy darstellt,
und R11 eine Gruppe ist, die aus den Gruppen (a) und (b) ausgewählt ist;
R2 ein Wasserstoffatom, ein Halogenatom oder einen der Reste (C₁-C₄)-Alkyl, (C₂-C₄)-Alkenyl, -O-(C₁-C₃)-Alkyl, -S-(C₁-C₃)-Alkyl, -(C₁-C₄)-Cycloalkyl, -CF₃, -NO₂, -CN oder -SO₃ darstellt;
R3 -(L₃)-Z darstellt, worin -(L₃)- eine zweibindige Gruppe ist, die aus einer Bindung und den Gruppen -CH₂-, -O-, -S-, -NH- und -CO- ausgewählt ist;
und Z aus den Gruppen Acetamid, Thioacetamid, Glyoxylamid, Thioglyoxylamid, Hydrazid oder Thiohydrazid ausgewählt ist, die durch die allgemeinen Formeln 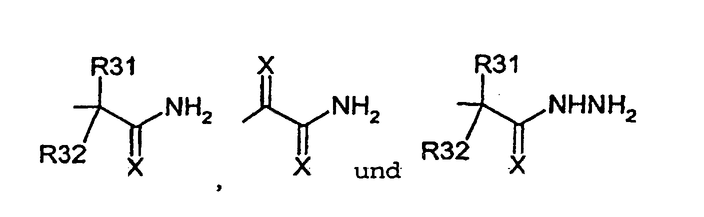 dargestellt werden, in denen R31 und R32 unabhängig voneinander aus einem Wasserstoffatom und einem der Reste (C₁-C₈)-Alkyl, (C₁-C₈)-Halogenalkyl oder (C₃-C₄)-Cycloalkyl ausgewählt sind und X jedesmal, wenn es auftritt, unabhängig voneinander ein Sauerstoffatom oder ein Schwefelatom darstellt;
R4 und R5 unabhängig voneinander aus einem Wasserstoffatom, einem nicht störenden Substituenten oder der Gruppe -(Lₐ)-(Acylsulfonamidgruppe) ausgewählt sind, in der -(Lₐ)-bei R4 eine Gruppe ist, die durch die Formel 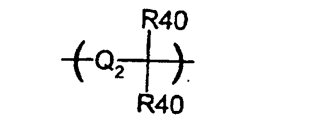 dargestellt ist, in der Q₂ aus -CH₂-, -O-, -NH-, -C(O)-und -S- ausgewählt ist und jede der Gruppen R40 unabhängig voneinander aus einem Wasserstoffatom, einem Halogenatom und den Resten (C₁-C₈)-Alkyl, Aryl, (C₁-C₈)-Alkaryl, (C₁-C₈)-Alkoxy und Aralkyl ausgewählt ist, -(Lₐ)- bei R5 aus den Gruppen ausgewählt ist, die durch die Formeln 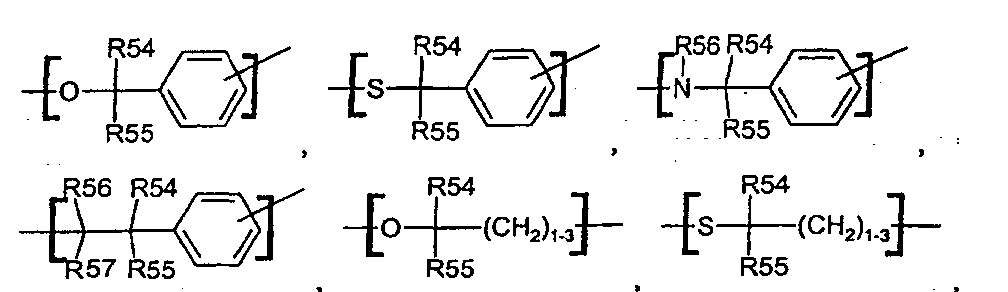 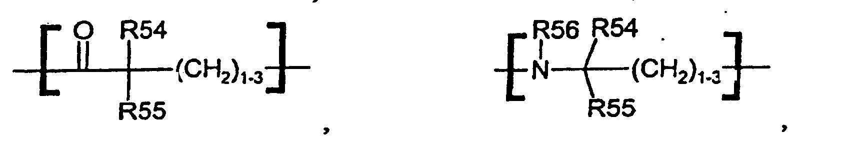 und 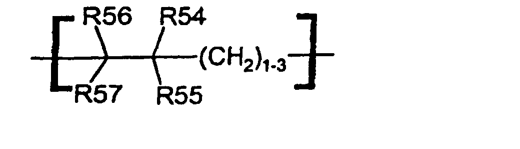 dargestellt werden, in denen R54, R55, R56 und R57 unabhängig voneinander aus der Gruppe ausgewählt sind, die aus einem Wasserstoffatom, einem Halogenatom und den Resten (C₁-C₈)-Alkyl, (C₁-C₈)-Alkoxy, (C₁-C₈)-Halogenalkyl und Aryl besteht,
wobei die Gruppe (Acylsulfonamid) eine Gruppe der Formel -CO-NH-SO₂-R81 ist, in der R81 aus der Gruppe ausgewählt ist, die aus den Resten -CF₃, (C₁-C₈)-Alkyl, (C₁-C₈)-Alkoxy, (C₁-C₈)-Alkylthio, (C₁-C₈)-Alkylamino, (C₁-C₈)-Halogenalkyl, (C₁-C₁₄)-Aralkyl, (C₁-C₁₄)-Alkylaryl, Aryl, Thioaryl, (C₃-C₁₄)-Cycloalkyl und einem Heterocyclus mit 3 bis 14 Kettengliedern besteht, wobei dieser Heterocyclus 1 bis 3 Heteroatome aufweist und gesättigt oder ungesättigt ist, wobei ferner gilt, daß mindestens einer von R4
und R5 eine Gruppe -(Lₐ)-(Acylsulfonamidgruppe) ist;
R6 und R7 unabhängig voneinander ein Wasserstoffatom, einen nicht störenden Substituenten, einen gesättigten oder ungesättigten kohlenstoffhaltigen Ring mit 5 bis 14 Kettengliedern, der ggf. durch einen oder mehrere Substituenten substituiert ist, die aus den nicht störenden Substituenten ausgewählt ist, oder einen Heterocyclus mit 5 bis 14 Kettengliedern darstellen, der 1 bis 3 Heteroatome enthält und gesättigt oder ungesättigt ist und ggf. durch einen oder mehrere Substituenten substituiert ist, die aus den nicht störenden Substituenten ausgewählt sind;
wobei die nicht störenden Substituenten unabhängig voneinander aus der Gruppe ausgewählt sind, die aus den Resten (C₁-C₈)-Alkyl, (C₂-C₈)-Alkenyl, (C₂-C₈)-Alkinyl, (C₇-C₁₂)-Aralkyl, (C₇-C₁₂)-Alkaryl, (C₃-C₈)-Cycloalkyl, (C₃-C₈)-Cycloalkenyl, Phenyl, Toluyl, Xylenyl, Biphenyl, (C₁-C₈)-Alkoxy, (C₂-C₈)-Alkenyloxy, (C₂-C₈)-Alkinyloxy, (C₂-C₁₂)-Alkoxyalkyl, (C₂-C₁₂)-Alkoxyalkyloxy, (C₂-C₁₂)-Alkylcarbonyl, (C₂-C₁₂)-Alkylcarbonylamino, (C₂-C₁₂)-Alkoxyamino, (C₂-C₁₂)-Alkoxyaminocarbonyl, (C₁-C₁₂)-Alkylamino, (C₁-C₆)-Alkylthio, (C₂-C₁₂)-Alkylthiocarbonyl, (C₁-C₈)-Alkylsulfinyl, (C₁-C₈)-Alkylsulfonyl, (C₂-C₈)-Halogenalkoxy, (C₁-C₈)-Halogenalkylsulfonyl, (C₂-C₈)-Halogenalkyl, (C₁-C₈)-Hydroxyalkyl, -C(O)O((C₁-C₈)-Alkyl), -(CH₂)ₙ-O-((C₁-C₈)-Alkyl), Benzyloxy, Phenoxy, Phenylthio, -CONHSO₂R, -CHO, Amino, Amidino, Brom, Carbamoyl, Carboxyl, Carbalkoxy, - (CH₂)ₙ-CO₂H, Chlor, Cyan, Cyanoguanidinyl, Fluor, Guanidino, Hydrazid, Hydrazino, Hydrazido, Hydroxy, Hydroxyamino, Iod, Nitro, Phosphono, -SO₃H, Thioacetal, Thiocarbonyl besteht, wobei n eine ganze Zahl von 1 bis 8 ist und R ein Rest (C₁-C₈)-Alkyl ist;
• den Verbindungen der allgemeinen Formel IV_{B} 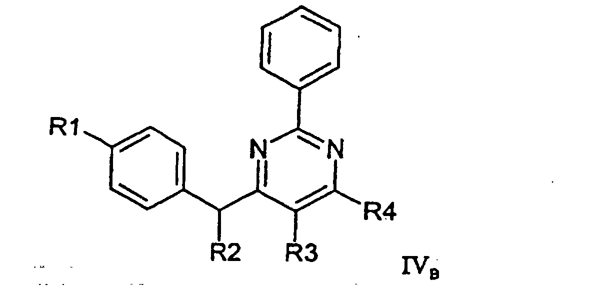 in der
R1 aus der Gruppe ausgewählt ist, die aus einem Halogenatom und den Resten (C₁-C₆)-Alkyl oder (C₁-C₆)-Alkoxy besteht;
R2 aus der Gruppe ausgewählt ist, die aus einem der Reste Phenyl oder Benzyl, die ggf. an dem aromatischen Ring durch ein Halogenatom oder einen der Reste (C₁-C₆)-Alkyl, (C₁-C₆)-Alkoxy, Amino, Alkylamino, Dialkylamino, Carboxyl, Carbamoyl, (C₁-C₈)-Acyl, Sulfonyl, Thiol oder Alkylthio substituiert sind, und aus einem Rest (C₃-C₇)-Cycloalkyl besteht,
wobei dieser Rest (C₃-C-₇)-Cycloalkyl ggf. an einen Benzolring oder einen Heterocyclus kondensiert ist und ggf. durch ein Halogenatom oder einen Rest (C₁-C₆)-Alkyl substituiert ist;
R3 aus der Gruppe ausgewählt ist, die aus einem Halogenatom und einem Rest (C₁-C₆)-Alkyl besteht;
und R4 aus der Gruppe ausgewählt ist, die aus -H, -OH, -N₃ und -NHCOCH₃ besteht;
• und den pharmazeutisch verträglichen Salzen der Verbindungen der allgemeinen Formel I_{B}, II_{B}, III_{B} oder IV_{B}.

9. Verwendung nach Anspruch 1, **dadurch gekennzeichnet, daß**
• der oder die NO-Synthasen-Hemmer aus der Gruppe ausgewählt sind, die aus L-Nitroarginin (LNA), L-Nitroarginin-Methylester (LNAME), L-N-Monomethylarginin (LNMMA), Aminoguanidin, Agmatin, 2-Amino-1-(methylamino)benzimidazol, 5-Nitroindazol, 6-Nitroindazol, 7-Nitroindazol, 1,2-(Trifluormethylphenyl)imidazol (TRIM), 2-Amino-4-methyl-6-(2-aminoethyl)pyridin, 2-Iminopiperidin, 2-Iminohomopiperidin, 2-Imino-5,6-dihydro-1,3-thiazin, 2-Imino-5,6-dihydro-1,3-oxazin, N-Phenyl-2-thiophencarboximidamid, 2-Iminotetrahydropyrimidin, S-Ethylisothioharnstoff, S-Methyl-L-thiocitrullin, S-Ethyl-L-thio-citrullin, (S)-N-{4-[4-[(3,4-Dihydro-6-hydroxy-2,5,7,8-tetramethyl-2H-1-benzopyran-2-yl)-carbonyl]-1-piperazinyl]phenyl}-2-thiophencarboximidamid und 4-[3,5-Bis(1,1-dimethylethyl)-4-hydroxyphenyl]-N-{4-[(imino(2-thienyl)methyl)amino]phenyl}-N-methyl-2-thiazolmethanamin sowie den pharmazeutisch verträglichen Salzen davon besteht; und
• der oder die Phospholipasen-A2-Hemmer aus der Gruppe ausgewählt sind, die aus Mepacrin und Aristolochiasäure und ihren Analogen sowie den pharmazeutisch verträglichen Salzen davon besteht.

10. Verwendung nach Anspruch 9, **dadurch gekennzeichnet, daß**
• der oder die NO-Synthasen-Hemmer aus der Gruppe ausgewählt sind, die aus Aminoguanidin, 7-Nitroindazol und 4-[3,5-Bis(1,1-dimethylethyl)-4-hydroxyphenyl]-N-{4-[(imino (2-thienyl)methyl)amino]phenyl}-N-methyl-2-thiazolmethanamin sowie den pharmazeutisch verträglichen Salzen der genannten Verbindungen besteht; und
• der oder die Phospholipasen-A2-Hemmer aus der Gruppe ausgewählt sind, die aus Mepacrin und Aristolochiasäure und ihren Analogen sowie aus den pharmazeutisch verträglichen Salzen davon besteht.

11. Verwendung nach einem der Ansprüche 1 bis 10, **dadurch gekennzeichnet, daß** die Krankheiten oder Störungen aus den proliferativen und entzündlichen Krankheiten, wie z.B. Atherosklerose, pulmonale Hypertension, Glomerulonephritis, portale Hypertension, Psoriasis, Arthrose und rheumatoide Arthritis, Fibrosen, Amyloidosen und Entzündungen des Magen-Darm-Systems oder des Lungensystems und der Atemwege, ausgewählt sind.

12. Verwendung nach einem der Ansprüche 1 bis 10, **dadurch gekennzeichnet, daß** die Krankheiten oder Störungen aus den Autoimmun- und Viruserkrankungen, wie z.B. Lupus, AIDS, parasitären und viralen Infektionen, Diabetes, Plaque-Sklerose oder Myopathien, ausgewählt sind.
